# EUROPÄISCHE PATENTANMELDUNG

(11) **EP 3 978 065 A1**
(43) Veröffentlichungstag der Anmeldung: **06.04.2022**
(21) Anmeldenummer: 20199745.9
(22) Anmeldetag: 02.10.2020
(51) Int. Cl.: A61M 39/02, A61M 39/04, A61M 5/142

(54) **IMPLANTAT ZUR LOKALEN WIRKSTOFFFREISETZUNG**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(57) **Zusammenfassung**

Die Erfindung betrifft ein Implantat zur lokalen Wirkstofffreisetzung aufweisend eine obere Wandung (1), die geschlossen und scheibenförmig ist und aus einem Material besteht, das mit einer Injektionskanüle mit einer Kraft von weniger als 100 N durchstechbar ist, selbstdichtend ist und elastisch verformbar ist, eine untere Wandung (2), die der oberen Wandung (1) gegenüberliegend angeordnet ist, scheibenförmig ist, elastisch verformbar ist und die zumindest eine flüssigkeitsdurchlässige Durchführung (3) durch die untere Wandung (2) aufweist, einen Hohlraum (4), der zwischen der oberen Wandung (1) und der unteren Wandung (2) angeordnet ist, und einen Durchstechschutz (6) zwischen der oberen Wandung (1) und der unteren Wandung (2), der scheibenförmig ist, wobei der Durchstechschutz (6) aus einem Material besteht, das mit einer Kraft von weniger als 100 N nicht mit einer Injektionskanüle (100) durchstechbar ist.

Die Erfindung betrifft auch ein Verfahren zum Befüllen eines solchen Implantats mit einer Flüssigkeit.

## Beschreibung

Die Erfindung betrifft ein Implantat zur lokalen Wirkstofffreisetzung und ein Verfahren zum Befüllen eines solchen Implantats mit einer Flüssigkeit.

Gegenstand der Erfindung ist somit ein Implantat, das zur lokalen Wirkstofffreisetzung, insbesondere zur lokalen Freisetzung von Immunsuppressoren, bestimmt ist. Das Implantat kann vom medizinischen Anwender nach erfolgter Implantation mehrfach mit Wirkstoffen neu beladen werden, ohne dass das Implantat dazu explantiert werden muss.

Rheumatische Erkrankungen gehören zu den sogenannten Autoimmunerkrankungen, bei denen das Immunsystem aus gegenwärtig noch nicht genau bekannten Ursachen körpereigene Strukturen angreift. Die Erkrankungen rheumatoide Arthritis und die Psoriasisarthritis gehören zum rheumatischen Formenkreis und sind mit Gelenkentzündungen (Arthritiden) verbunden. Diese meist chronisch verlaufenden Gelenkentzündungen können zur fortschreitenden Zerstörung der Gelenke führen. Die Gelenke des Menschen sind von einer Gelenkkapsel (Capsula articulatis) umgeben. Diese setzt sich aus einer äußeren Bindegewebsschicht (Membrana fibrosa) und einer inneren Schicht der sogenannten Synovialmembran (Membrana synovialis) zusammen. Die Synovialmembran ist für die Entzündungsprozesse bei der rheumatoiden Arthritis und der Psoriasisarthritis entscheidend, weil durch diese hindurch fehlgesteuerte Immunzellen einwandern, beziehungsweise weil durch die Proliferation von Fibroblasten auf der Synovialmembran ein Pannus - das ist ein Zellklon von Fibroblasten - gebildet werden kann. Beim Zerfall des Pannus und durch die eingewanderten Immunzellen werden Entzündungsmediatoren wie Interleukine und α-TNF (a-Tumornekrosefaktor) sezerniert. Diese Entzündungsmediatoren steuern über eine Entzündungskaskade die Freisetzung von Proteasen und anderen destruierenden Enzymen aus Immunzellen, welche dann zu einer enzymatischen Zerstörung der Knorpel- und Knochenstrukturen führen. Exemplarisch seien dafür die folgenden Publikationen genannt:
"Analysis of the inflammatory cytokine network among TNF alpha, IL-1 beta, IL-1 receptor antagonist, and IL-8 in LPS-induced rabbit arthritis", Matsukawa A., Yoshimura T., Miyamoto K., Ohkawara S., Yoshinaga M., Lab Invest. 1997 May;76(5):629-38. PMID: 9166282.;
"Cytokine concentrations in the synovial fluid and plasma of rheumatoid arthritis patients: correlation with bony erosions", Fong K.Y., Boey M.L., Koh W.H., Feng P.H., Clin. Exp. Rheumatol. 1994 Jan-Feb;12(1):55-8. PMID: 8162643.;
"Study of pro-inflammatory (TNF-alpha, IL-1alpha, IL-6) and T-cell-derived (IL-2, IL-4) cytokines in plasma and synovial fluid of patients with juvenile chronic arthritis: correlations with clinical and laboratory parameters", Kutukculer N., Caglayan S., Aydogdu F., Clin. Rheumatol. 1998;17(4):288-92. doi: 10.1007/BF01451007. PMID: 9776110.;
"Preliminary report on cytokine determination in human synovial fluids: a consensus study of the European Workshop for Rheumatology Research", The Cytokine Consensus Study Group of the European Workshop for Rheumatology Research. Roux-Lombard P., Steiner G., Clin. Exp. Rheumatol. 1992 Sep-Oct; 10(5):515-20. PMID: 1333926 Review.; und
"The role of T-cell interleukin-17 in conducting destructive arthritis: lessons from animal models", Lubberts E., Koenders M.I., van den Berg W.B., Arthritis Res. Ther. 2005; 7(1):29-37. doi: 10.1186/ar1478. Epub 2004 Nov 30. PMID: 15642151 Free PMC article. Review.

Für die systemische medikamentöse Therapie der rheumatoiden Arthritis und die Psoriasisarthritis stehen eine Reihe von Wirkstoffgruppen zur Verfügung. Diese Wirkstoffgruppen werden in Analgetika, nicht-steroidale Antiphlogistika, Glucocorticoide und Basistherapeutika (Disease modifying anti-rheumatic drugs, DMARD) unterteilt. Daneben gibt es moderne Antikörper (Biologicals), welche das α-TNF (a-Tumornekrosefaktor) oder das Interleukin IL-17 blockieren. Besonders interessant sind niedermolekulare Wirkstoffe, die das Immunsystem supprimieren. Wichtige Wirkstoffe sind hierbei das Cyclosporin A (CAS 59865-13-3), das Tacrolimus (CAS 104987-11-3) und Everolimus (CAS 159351-69-6).

Der Wirkungsmechanismus von Cyclosporin A ist auf die Bildung eines Komplexes aus Cyclosporin A und dem Immunophilin Cyclophilin zurückzuführen. Dieser Komplex inhibiert die Phosphatase Calcineurin. Das Enzym Calcineurin ist wiederum essentiell für die Bildung der α -TNF, wobei die α-TNF eine Schlüsselverbindung am Beginn der Entzündungskaskaden ist. Durch Inhibieren der Synthese von α-TNF können demzufolge die nachfolgenden Entzündungskaskaden ebenfalls inhibiert werden.

Bei der systemischen medikamentösen Therapie können Serum-Wirkstoffkonzentrationen für Cyclosporin A im Bereich von ca. 75 µg/l bis 175 µg/l und für Tacrolimus von 5 µg/l bis 20 µg/l erreicht werden. Höhere systemische Wirkstoffkonzentrationen sind auf Grund der Gefahr von unerwünschten toxischen Nebenwirkungen für den restlichen Organismus systemisch für eine länger andauernde Therapie nicht möglich. Das bedeutet, dass im Gewebe der Gelenkskapsel (Membrana fibrosa und Membrana synovialis) die Wirkstoffkonzentrationen sehr niedrig sind und daher nur einen begrenzten immunmodulierenden Effekt auf das Entzündungsgeschehen haben können.

Zur Verminderung der chronischen Entzündungsprozesse im Gelenkbereich von Patienten mit rheumatoider Arthritis und mit Psoriasisarthritis wäre es daher wünschenswert, wenn Immunsuppressoren direkt am Gewebe der Gelenkskapsel appliziert werden könnten, um eine lokal hohe Konzentration dieser Immunsuppressoren zu erreichen.

Es sind bereits Implantate zur Abgabe von pharmazeutischen Wirkstoffen im Bereich der Gelenke bekannt, die zur lokalen Applikation von Antibiotika eingesetzt werden. Beispielhaft seien hierfür die Dokumente GB 2 290 971 A, US 2010/0042213 A, WO 2017/178951 A1, WO 2007/084878 A1 und US 6 245 111 B1 genannt. Bei diesen Implantaten ist aber das Gelenk bereits großflächig entfernt worden und die Implantate dienen vornehmlich der Behandlung der Prothesen mit Antibiotika. Derartige Implantate zur Behandlung einer rheumatoiden Arthritis oder einer Psoriasisarthritis einzusetzen kommt nicht in Betracht, weil das zu behandelnde Gelenk für die Therapie entfernt werden müsste und dadurch die nachfolgende Behandlung sinnlos wäre. Die US 2007/0219471 A1 offenbart ein Implantat zum Applizieren von Wirkstoffen und Aufrechterhalten eines reduzierten Drucks zur Beschleunigung der Wundheilung in und zur mechanischen Stabilisierung von schlecht heilenden Kavitäten.

Aus der WO 2010/088548 A1 ist ein röhrenförmiges, wiederauffüllbares Augen-Implantat bekannt, mit dem ein pharmazeutischer Wirkstoff direkt an den Augapfel abgegeben werden kann. Hierzu wird das röhrenförmige Implantat direkt in den Augapfel eingesteckt. Für eine Implantation in ein Gelenk ist das Implantat nicht vorgesehen und auch nicht geeignet. Durch das Einsetzen im Gelenk würde das Gelenk selbst angegriffen und beschädigt und bei Einsetzen des Implantats bestünde das Risiko einer Infektion und Entzündung des Gelenks.

Die US 5 681 289 A und die US 2018/0028320 A1 offenbaren Implantate, bei denen eine medizinische Flüssigkeit zur Behandlung von Gelenken oder zum Schmieren von Gelenken aus einem Reservoir über Schläuche den Gelenken zugeführt wird. Die Implantate sind relativ groß und nur aufwendig zu platzieren. Eine kurzfristige Änderung einer medizinischen Behandlung ist aufwendig, weil die Zuleitungen zu den flächenförmigen Applikatoren zuerst gespült werden müssten, was im implantierten Zustand nicht ohne weiteres möglich ist. Für das Schmieren von Gelenken mit einem über das Implantat abgegebenen Schmiermittel sind die Implantate gut geeignet, während eine gezielte Abgabe von pharmazeutischen Wirkstoffen mit hoher Konzentration mit Hilfe dieser Implantate nicht möglich erscheint, weil in dem Implantat, dem Reservoir und den Zuleitung zu große Volumina enthalten sind.

In der US 2003/0139811 A1 ist ein Wirkstoffsystem zur lokalen Freisetzung von entzündungshemmenden Wirkstoffen im Innenraum von Gelenkskapseln offenbart. Dabei handelt es sich um hohle Schrauben mit einer Öffnung im Schraubenkopf. Diese hohlen Schrauben können mit einem Wirkstoff befüllt werden. Anschließend werden die Schrauben in Knochengewebe eingebracht, das sich neben dem Knorpelgewebe innerhalb der Gelenkskapsel befindet. Über die Öffnung im Schraubenkopf soll anschließend der Wirkstoff in die Synovialflüssigkeit freigesetzt werden. Kritisch ist hierbei zu sehen, dass die Gelenkskapsel eröffnet werden muss und dass die Schraube nach erfolgter Wirkstofffreisetzung im Innenraum der Gelenkskapsel verbleibt. Eine Eröffnung der Gelenkskapsel ist immer mit einem deutlichen Infektionsrisiko behaftet. Eine Auffüllung der implantierten Schraube mit neuem Wirkstoff ist nicht vorgesehen. Daher kann die Behandlung auch nicht an eine Veränderung des Zustands des behandelten Patienten angepasst werden.

Der Erfindung liegt die Aufgabe zugrunde, die Nachteile des Stands der Technik zu überwinden. Insbesondere soll ein Implantat zur lokalen Freisetzung von Wirkstoffen bereitgestellt werden, mit dem eine lokale Behandlung von Immunsuppressoren mit hoher Konzentration auch über längere Zeiträume möglich ist. Die Behandlung soll dabei möglichst auch kurzfristig an die individuelle Behandlungssituation eines Patienten anpassbar sein.

Aufgabe der Erfindung ist es somit auch, ein subkutanes Implantat zu entwickeln, das zur lokalen Wirkstofffreisetzung geeignet ist. Es soll bevorzugt möglich sein, dass das im Patienten befindliche Implantat vom medizinischen Anwender auf einfache Weise mit frischen und wechselnden Wirkstoffen bestückt werden kann, ohne dass dazu das Implantat explantiert werden muss. Es muss sichergestellt werden, dass der medizinische Anwender bei der Verwendung des Implantats nicht die mit dem Implantat zu behandelnden Gewebestrukturen schädigen oder verletzen kann. Das Implantat soll durch Röntgen im Patienten dargestellt werden können.

Die Aufgaben der Erfindung werden gelöst durch ein Implantat zur lokalen Wirkstofffreisetzung, das Implantat aufweisend
eine obere Wandung, wobei die obere Wandung geschlossen und scheibenförmig ist und aus einem Material besteht, das mit einer medizinischen Injektionskanüle mit einer Kraft von weniger als 100 N durchstechbar ist, das selbstdichtend ist und das elastisch verformbar ist, eine untere Wandung, wobei die untere Wandung der oberen Wandung gegenüberliegend angeordnet ist, scheibenförmig ist, elastisch verformbar ist und die untere Wandung zumindest eine Durchführung durch die untere Wandung aufweist, wobei die zumindest eine Durchführung flüssigkeitsdurchlässig ist,
einen Hohlraum, der zwischen der oberen Wandung und der unteren Wandung angeordnet ist, und
einen Durchstechschutz, wobei der Durchstechschutz zwischen der oberen Wandung und der unteren Wandung angeordnet ist und scheibenförmig ist, wobei der Durchstechschutz aus einem Material besteht, das mit einer Kraft von weniger als 100 N nicht mit einer medizinischen Injektionskanüle durchstechbar ist.

Das Implantat ist vorzugsweise ein medizinisches Implantat. Alle Materialien des Implantats, die mit dem Körper beziehungsweise Gewebe des Körpers in Kontakt kommen können, sollten daher besonders bevorzugt biokompatibel sein. Die Materialien, aus denen die obere Wandung und die untere Wandung bestehen, sind also vorzugsweise biokompatibel.

Das Implantat soll bevorzugt im Bereich der Außenseiten von Gelenkkapseln im Fall von chronischen durch Autoimmunprozesse verursachten Gelenkentzündungen implantiert werden und ist dann demzufolge hierfür vorgesehen und geeignet. Exemplarisch sind dafür aus dem rheumatischen Formenkreis die chronische rheumatoide Arthritis und die Psoriasisarthritis. Besonders bevorzugt ist das Implantat also ein Implantat zur Behandlung von chronischer rheumatoider Arthritis und Psoriasisarthritis.

Unter einer medizinischen Injektionskanüle wird eine Injektionskanüle einer beliebigen handelsüblichen Spritze aus dem medizinischen Bereich verstanden und kein Spezialwerkzeug zum Durchstechen besonders harter Materialien. Solche Injektionskanülen können beispielsweise durch die Normen ISO 7864:2016 (2016-08) und DIN 13097-4 (1. Juni 2019) charakterisiert sein. Beispiel hierfür sind Sterican^{®} Standardkanülen der Firma B. Braun Melsungen AG.

Selbstdichtend bedeutet, dass die obere Wandung, nachdem sie von einer medizinischen Injektionskanüle durchstochen wurde und die medizinische Injektionskanüle wieder aus der oberen Wandung herausgezogen wurde, sich selbsttätig wieder flüssigkeitsdicht verschließt.

Bevorzugt kann vorgesehen sein, dass das Implantat flach und/oder scheibenförmig ist.

Ein scheibenförmiger Körper hat eine geringere Dicke beziehungsweise Stärke als seine Breite und Tiefe und hat eine im Wesentlichen ebene Form.

Das Implantat kann sowohl vor als auch nach der Implantation mit einer Flüssigkeit und damit mit zumindest einen Wirkstoff gefüllt beziehungsweise beladen werden. Das Implantat kann dann durch die zumindest eine Durchführung die Flüssigkeit beziehungsweise den zumindest einen Wirkstoff abgeben.

Bevorzugt kann das Implantat zur lokalen Freisetzung von Immunsuppressoren geeignet und bestimmt sein. Das Implantat kann vom medizinischen Anwender nach erfolgter Implantation mehrfach mit Wirkstoffen neu beladen werden, ohne dass das Implantat dazu explantiert werden muss.

Es kann vorgesehen sein, dass das Implantat und/oder die obere Wandung und/oder die untere Wandung eine kreisförmige Form hat oder haben. Diese Geometrie weist bei flachem Aufbau das beste Volumen zu Oberflächenverhältnis auf und lässt sich gut an Gelenken implantieren.

Es kann vorgesehen sein, dass die obere Wandung und die untere Wandung über einen umlaufenden Rand miteinander verbunden sind oder über eine umlaufende Begrenzung des Durchstechschutzes miteinander verbunden sind, wobei bevorzugt die obere Wandung und die untere Wandung flüssigkeitsdicht miteinander verbunden sind.

Hierdurch ergibt sich ein kompakter und flacher Aufbau des Implantats, so dass dieses gut auf einer Außenseite einer Gelenkkapsel durch Implantation aufgelagert werden kann. Wenn die obere Wandung und die untere Wandung flüssigkeitsdicht miteinander verbunden sind, kann die Flüssigkeit aus dem Hohlraum nur durch die zumindest eine Durchführung aus dem Implantat abgegeben werden. Bevorzugt sind die obere Wandung und die untere Wandung über einen umlaufenden Rand der oberen Wandung und einen umlaufenden Rand der unteren Wandung direkt miteinander verbunden. Der umlaufende Rand kann also zur oberen Wandung und/oder zur unteren Wandung gehören, insbesondere also auch zweiteilig sein und sowohl zur oberen Wandung als auch zur unteren Wandung gehören. Es kann auch vorgesehen sein, dass die obere Wandung und die untere Wandung durch einen Rastmechanismus miteinander verbunden sind, insbesondere flüssigkeitsdicht miteinander verbunden sind.

Alternativ können die obere Wandung und die untere Wandung aber auch einteilig aufgebaut sein. Dies hat insbesondere bei der Herstellung sehr kleiner Implantate Vorteile. Zur Herstellung kann beispielsweise der Durchstechschutz in eine elastische oder gummielastische Blase eingelegt werden und die untere Wandung und die obere Wandung anschließend mit einem Blasform-Verfahren geformt werden. Hierzu kann die Grundform, in die der Durchstechschutz eingelegt ist, mit einem Gasdruck aufgeblasen und an eine äußere Form gedrückt werden und anschließend in diesem Zustand verfestigt werden.

Des Weiteren kann vorgesehen sein, dass der Durchstechschutz aus Metall besteht oder zu mindestens 50% aus Metall besteht.

Hierdurch kann ein Durchstechen des Durchstechschutzes zuverlässig verhindert werden. Zudem können Metalle besonders gut im medizinischen Bereich eingesetzt werden, da es viele biokompatible Metalle und Metalllegierungen gibt. Ferner lässt sich dadurch der Durchstechschutz gut mit Röntgenstrahlung darstellen. Die Position des Implantats im Patienten kann somit leicht kontrolliert werden. Als Metall kommen bevorzugt Edelstahl, Titan, Titanlegierungen, Tantal, Tantallegierungen und auch Silber enthaltende Legierung als auch Silber in Betracht.

Ferner kann vorgesehen sein, dass der Durchstechschutz in dem Hohlraum angeordnet ist, wobei bevorzugt der Durchstechschutz nicht fest mit der oberen Wandung verbunden ist und besonders bevorzugt der Durchstechschutz auf einer der oberen Wandung zugewandten Seite der unteren Wandung angeordnet ist.

Hierdurch kann sich der Durchstechschutz während dem Einspritzen von Flüssigkeit in den Hohlraum von der oberen Wandung abheben und so der Hohlraum sich in diesem Bereich ausdehnen. Dadurch kann sichergestellt werden, dass auch bei flachem Aufbau des Durchstechschutzes die Öffnung der medizinischen Injektionskanüle im Inneren des Hohlraums über dem Durchstechschutz angeordnet ist und dadurch die Flüssigkeit im Inneren des Hohlraums eingespritzt werden kann.

Es kann auch vorgesehen sein, dass die untere Wandung mit einer medizinischen Injektionskanüle durchstechbar ist, wobei bevorzugt die untere Wandung aus einem gummielastischen Kunststoff besteht.

Hierdurch kann die untere Wandung aus einem hoch-elastischen Material wie Gummi gefertigt werden, so dass der Hohlraum auch im unteren Bereich dehnbar ist. Dadurch kann eine größere Menge der Flüssigkeit in den Hohlraum injiziert werden. Hierdurch kann sich zudem die Unterseite des Implantats, also die untere Wandung, an die Form der äußeren Oberfläche der Gelenkkapsel anpassen.

Gemäß einer bevorzugten Weiterbildung kann vorgesehen sein, dass der Hohlraum durch Injektion einer Flüssigkeit elastisch expandierbar ist, wobei bevorzugt die Flüssigkeit in dem expandierten Hohlraum mit einer elastischen Kraft durch die zumindest eine Durchführung aus dem Hohlraum auspressbar ist.

Hierdurch kann die Flüssigkeit aus dem Hohlraum durch einen Druck auf den Hohlraum, insbesondere durch die von der oberen Wandung und der unteren Wandung wirkende elastische Rückstellkraft, an die Umgebung herausgedrückt werden.

Des Weiteren kann vorgesehen sein, dass die untere Wandung auf einer der oberen Wandung gegenüberliegenden Seite eine abstehende Strukturierung aufweist, die zur Verteilung einer Flüssigkeit auf dieser äußeren Oberfläche geeignet ist, wobei bevorzugt die abstehende Strukturierung Stege und/oder Noppen sind und/oder die zumindest eine Durchführung in zumindest einen Kanal auf dieser äußeren Oberfläche der unteren Wandung mündet und der zumindest eine Kanal durch die abstehende Strukturierung geformt ist.

Hierdurch kann verhindert werden, dass die zumindest eine Durchführung verstopft wird oder sich durch eine Auflage verschließt. Zudem wird so erreicht, dass sich die Flüssigkeit aus dem Hohlraum gut an der äußeren Oberfläche der unteren Wandung verteilen kann. Das Implantat wird durch Weichgewebe überlagert. Dieses kann Druck auf die obere Wandung ausüben, wobei die obere Wandung dadurch Druck auf die Wirkstofflösung im Hohlraum ausüben kann. Eine Beschleunigung des Austritts der Wirkstofflösung durch die Durchführung in der unteren Wandung kann dadurch erreicht werden. Durch die abstehende Strukturierung beziehungsweise durch die Stege und/oder Noppen an der Unterseite der oberen Wandung wird eine Bewegung der oberen Wandung in Richtung der unteren Wandung verhindert. Dadurch wird der Innenraum und der Hohlraum druckentlastet und ein Auspressen der Wirkstofflösung aus dem Hohlraum verhindert.

Bevorzugt kann auch vorgesehen sein, dass das Verhältnis der Breite zur Höhe und der Tiefe zur Höhe des Implantats mindestens 2:1 ist, bevorzugt zumindest 3:1 ist.

Hierdurch kann das Implantat gut unter der Haut im Bereich von Gelenken implantiert werden. Zudem kann die obere Wandung auf diese Weise sehr leicht mit einer medizinischen Injektionskanüle getroffen werden, um den Hohlraum beziehungsweise den Innenraum mit einer Flüssigkeit zu füllen.

Auch kann vorgesehen sein, dass im Hohlraum eine Flüssigkeit enthalten ist, wobei die Flüssigkeit bevorzugt zumindest einen pharmazeutischen Wirkstoff enthält, wobei besonders bevorzugt einer des zumindest einen Wirkstoffs Cyclosporin A ist, oder im Hohlraum eine Wirkstofflösung oder ein im festen oder halbfesten Zustand befindlicher Wirkstoff enthalten ist.

Auf diese Weise ist das Implantat unmittelbar zur medizinischen Behandlung oder zu einer Flüssigkeitsabgabe einsetzbar oder nach Befüllen mit einer Trägerflüssigkeit beziehungsweise einem Lösungsmittel für die festen oder halbfesten Wirkstoffe zur medizinischen Behandlung oder zu einer Flüssigkeitsabgabe einsetzbar.

Ferner kann vorgesehen sein, dass der Durchstechschutz mindestens so groß ist wie 50% einer inneren Oberfläche der unteren Wandung, die den Hohlraum begrenzt, bevorzugt mindestens so groß ist wie 75% der inneren Oberfläche der unteren Wandung.

Hierdurch wird sichergestellt, dass nicht sehr genau mit der medizinischen Injektionskanüle getroffen beziehungsweise gezielt werden muss, um ein Durchstechen der unteren Wandung zu vermeiden.

Des Weiteren kann vorgesehen sein, dass der Durchstechschutz an einer umlaufenden Begrenzung des Durchstechschutzes nicht bündig an der Innenseite des Hohlraums anliegt.

Dadurch kann die Flüssigkeit in dem Hohlraum von dem oberen Innenraum zwischen dem Durchstechschutz und der oberen Wandung bis zum unteren Innenraum zwischen dem Durchstechschutz und der unteren Wandung fließen.

Es kann bevorzugt vorgesehen sein, dass das Implantat scheibenförmig ist und die obere Wandung eine Oberseite des Implantats bildet und die untere Wandung eine Unterseite des Implantats bildet, wobei bevorzugt die gesamte Oberfläche des Implantats oder die gesamte Oberfläche des Implantats bis auf einen umlaufenden Rand durch die obere Wandung und die untere Wandung gebildet ist.

Hierdurch kann ein kompaktes und flaches Implantat bereitgestellt werden, das leicht mit einer medizinischen Injektionskanüle einer Spritze zu treffen ist und so die Flüssigkeit leicht in den Hohlraum injizierbar ist.

Gemäß einer bevorzugten Weiterbildung kann vorgesehen sein, dass der Hohlraum einen Innenraum aufweist, der von dem Oberteil und von dem Durchstechschutz begrenzt ist, wobei bevorzugt durch den Innenraum das Oberteil von dem Durchstechschutz zumindest 0,5 mm beabstandet ist, besonders bevorzugt zumindest 1 mm beabstandet ist.

Hierdurch wird sichergestellt, dass eine Öffnung in einer medizinischen Injektionskanüle in dem Innenraum angeordnet ist, wenn eine medizinische Injektionskanüle durch das Oberteil gestochen wird. Dadurch kann sichergestellt werden, dass mit der medizinischen Injektionskanüle eine medizinische Flüssigkeit in den Innenraum und damit in den Hohlraum des medizinischen Implantats gespritzt werden kann.

Auch kann vorgesehen sein, dass in jeder der zumindest einen Durchführung jeweils ein Überdruckventil angeordnet ist, das ab einem Mindestdruck nach außen öffnet, bevorzugt ab einem Mindestdruck von mindestens 20 kPa nach außen öffnet.

Hierdurch kann die Flüssigkeit aus dem Hohlraum, und damit die Wirkstoffe in der Flüssigkeit, durch einen Druck auf den Hohlraum im Körper des Patienten gezielt appliziert beziehungsweise freigesetzt werden. Zudem kann so ein Rückfluss von Körperflüssigkeiten in das Implantat verhindert werden.

Ferner kann vorgesehen sein, dass die obere Wandung und/oder die untere Wandung aus einem gummielastischen Kunststoff besteht oder bestehen oder bis auf einen Rand aus einem gummielastischen Kunststoff besteht oder bestehen, wobei bevorzugt der gummielastische Kunststoff der oberen Wandung sich nach einem Durchstechen und wieder Herausziehen eine medizinischen Injektionskanüle wieder zusammenzieht und flüssigkeitsundurchlässig verschließt.

Hierdurch kann zum einen erreicht werden, dass die Flüssigkeit nicht aus der oberen Wandung austritt, und zum anderen kann durch die elastische Kraft des gummielastischen Kunststoffs die Flüssigkeit durch die zumindest eine Durchführung aus dem Hohlraum des Implantats ausgepresst werden.

Bevorzugt kann auch vorgesehen sein, dass die obere Wandung und die untere Wandung flüssigkeitsdicht sind, besonders bevorzugt auch Wirkstoffdicht sind, das heißt, dass ein in der Flüssigkeit enthaltener Wirkstoff nicht durch die obere Wandung und die untere Wandung hindurchdiffundieren kann.

Bevorzugt kann vorgesehen sein, dass die untere Wandung aus einem gummielastischen Kunststoff besteht oder zumindest in einem mittleren Bereich aus einem gummielastischen Kunststoff besteht, der mit Injektionskanülen durchstechbar ist, wobei besonders bevorzugt die Durchstechöffnung nach Entfernung der Injektionskanüle durch die Rückstellkraft des gummielastischen Kunststoffs wieder verschließbar ist.

Des Weiteren kann vorgesehen sein, dass der Durchstechschutz zumindest eine flüssigkeitsdurchlässige Durchbrechung aufweist, wobei bevorzugt die zumindest eine Durchbrechung einen freien Querschnitt von maximal 0,5 mm hat, besonders bevorzugt von maximal 0,25 mm hat.

Hierdurch kann die Flüssigkeit im Hohlraum von der einen Seite des Durchstechschutzes zur anderen Seite des Durchstechschutzes fließen beziehungsweise zu der zumindest einen Durchführung der unteren Wandung gelangen.

Des Weiteren kann auch vorgesehen sein, dass der Durchstechschutz auf der der unteren Wandung zugewandten Seite eine Oberflächenstrukturierung aufweist, bevorzugt auf der der oberen Wandung zugewandten Seite und auf der der unteren Wandung zugewandten Seite eine Oberflächenstrukturierung aufweist.

Hierdurch wird verhindert, dass die untere Wandung zu dicht an der ihr zugewandten Unterseite des Durchstechschutzes anliegt. Durch die Oberflächenstrukturierung auf der Oberseite des Durchstechschutzes kann erreicht werden, dass die Öffnung an der Spitze der medizinischen Injektionskanüle in jedem Fall im Hohlraum angeordnet ist, wenn die medizinische Injektionskanüle bis zum Durchstechschutz durch die obere Wandung eingestochen ist. Die Oberflächenstrukturierung kann bevorzugt durch Nuten oder auch durch kreisförmige Vertiefungen ausgebildet sein. An der Oberseite des Durchstechschutzes angeordneten Strukturierungen verhindern auch ein Abgleiten der medizinischen Injektionskanüle auf der Oberseite des Durchstechschutzes. Alternativ ist es auch möglich den Durchstechschutz an seiner Oberseite mit einer umlaufenden Begrenzung zu versehen. Die umlaufende Begrenzung kann ein nach oben vorstehender Rand sein. Dann ist auch kein Abrutschen der medizinischen Injektionskanüle möglich, was zu einer zweiten Perforation der oberen Wandung führen könnte. Ferner kann bevorzugt vorgesehen sein, dass der Durchstechschutz als leicht konkave Scheibe ausgeformt ist. Dadurch wird einem Abgleiten der medizinischen Injektionskanüle ebenfalls entgegengewirkt. Strukturierungen an der Unterseite des Durchstechschutzes können es ermöglichen, dass die Wirkstofflösung zu der zumindest einen Durchbrechung der unteren Wandung gelangen können.

Bevorzugt kann vorgesehen sein, dass der Durchstechschutz mit der unteren Wandung fest verbunden ist.

Hierdurch kann der Durchstechschutz relativ zur unteren Wandung, die er schützen soll, fixiert werden.

Ebenfalls kann vorgesehen sein, dass der Durchstechschutz auf einer Oberseite, die der oberen Wandung zugewandt ist, eine konkave Form hat und/oder eine vorstehende Begrenzung aufweist.

Hiermit kann verhindert werden, dass die Spitze der medizinischen Injektionskanüle ungewollt abgleitet. Zudem bildet sich so ein Innenraum in der konkaven Mulde und der oberen Wandung, in die die Flüssigkeit mit der medizinischen Injektionskanüle eingespritzt werden kann.

Ferner kann vorgesehen sein, dass das Implantat eine oder mehrere Laschen aufweist, an denen das Implantat an Weichgewebe angenäht werden kann.

Hierdurch kann das Implantat an Weichgewebe fixiert werden. Zusätzlich ist dadurch eine genaue Positionierung des Implantats möglich und ein Wandern des Implantats kann ausgeschlossen werden.

Die der vorliegenden Erfindung zugrundeliegenden Aufgaben werden auch gelöst durch ein Verfahren zum Befüllen eines solchen Implantats mit den Schritten:
A) Bereitstellen des Implantats und einer mit einer Flüssigkeit gefüllten Spritze, wobei die Spritze eine medizinische Injektionskanüle aufweist,
B) Durchstechen der oberen Wandung mit der medizinischen Injektionskanüle der Spritze,
C) Einspritzen von Flüssigkeit aus der Spritze durch die medizinische Injektionskanüle in den Hohlraum des Implantats,
D) Ausbreiten der Flüssigkeit in dem Hohlraum, wobei die Flüssigkeit bis zu der zumindest einen Durchführung fließt, und,
E) optional, elastisches Expandieren des Hohlraums durch Injizieren der Flüssigkeit.

Die Flüssigkeit enthält bevorzugt zumindest einen pharmazeutischen Wirkstoff. Der Schritt E) ist erfindungsgemäß bevorzugt aber optional.

Es kann vorgesehen sein, dass bei dem Verfahren keine medizinische Behandlung eines menschlichen oder tierischen Körpers erfolgt und/oder die Flüssigkeit im Rahmen des Verfahrens nicht an einen menschlichen oder tierischen Körper abgegeben wird.

Hiermit wird klargestellt, dass es sich bei dem erfindungsgemäßen Verfahren nicht um ein Verfahren zur Behandlung des menschlichen Körpers handelt.

Des Weiteren kann vorgesehen sein, dass in Schritt B) die Spitze der medizinischen Injektionskanüle derart weit durch die obere Wandung gestochen wird, dass sie auf den Durchstechschutz trifft, wobei eine Öffnung an der Spitze der medizinischen Injektionskanüle dann in einem Innenraum des Hohlraums liegt, wobei der Innenraum des Hohlraums zwischen dem Durchstechschutz und der oberen Wandung angeordnet ist.

Hierdurch kann sichergestellt werden, dass die Flüssigkeit mit der medizinischen Injektionskanüle in den Hohlraum eingespritzt werden kann.

Bevorzugt kann vorgesehen sein, dass nach Schritt E) die folgenden Schritte F) und G) und gegebenenfalls H) erfolgen:
F) Herausziehen der medizinischen Injektionskanüle aus dem Hohlraum und aus der oberen Wandung und
G) flüssigkeitsdichtes Verschließen der oberen Wandung durch gummielastische Rückstellung der oberen Wandung, und optional
H) Komprimieren des Hohlraums aufgrund der elastischen Rückstellkraft des elastisch expandierten Hohlraums und Herausdrücken der Flüssigkeit aus dem Hohlraum durch die zumindest eine Durchführung.

Damit kann sichergestellt werden, dass die medizinische Flüssigkeit nur durch die zumindest eine Durchführung appliziert wird und nicht ungewollt auf der gegenüberliegenden Oberseite des Implantats austritt.

Der Erfindung liegt die überraschende Erkenntnis zugrunde, dass es durch den Durchstechschutz in der Mitte zwischen zwei elastischen Wandungen gelingt, ein kompaktes Implantat bereitzustellen, dass einen flachen Aufbau hat und somit für den subkutanen Einsatz im Bereich von Gelenken geeignet ist, wobei das Implantat in einer Wandung mit einer medizinischen Injektionskanüle durchstechbar ist, so dass das Implantat auf einfache Weise wiederholt durch die Haut hindurch gefüllt werden kann, und das Implantat an seiner gegenüberliegenden Wandung zumindest eine Durchführung hat, durch die die Flüssigkeit enthaltend den Wirkstoffe oder die Wirkstoffe direkt an die äußere Gelenkkapseloberfläche oder an die zu behandelnde Stelle in hoher Konzentration abgegeben werden kann. Durch den elastisch expandierbaren Hohlraum gelingt es dabei, die Flüssigkeit aus dem Hohlraum des Implantats kontinuierlich abzugeben. Durch die scheibenförmige untere Wandung, die implantiert auf dem Gelenk als Auflage aufliegt, kann eine flächige Kontaktierung und eine gefächerte Verteilung des Wirkstoffs am Gelenk erreicht werden. Aufgrund der elastischen Verformbarkeit des Implantats kann das Implantat zumindest etwas an die Form der Oberfläche des zu behandelnden Gelenks angepasst werden. Der Durchstechschutz verhindert dabei, dass das Implantat versehentlich durchstochen werden kann und es so eine Verletzung des darunter liegenden Gewebes kommt. Zudem kann so verhindert werden, dass die Flüssigkeit aus Versehen nicht innerhalb des Implantats appliziert wird. Mit dem erfindungsgemäßen Implantat wird erreicht, dass das im Patienten befindliche Implantat vom medizinischen Anwender mit Hilfe üblicher medizinischer Injektionskanülen einfacher Spritzen mehrfach aufgefüllt werden kann, ohne dass dazu das Implantat explantiert werden muss. Dabei ist mit dem Durchstechschutz sichergestellt, dass der medizinische Anwender mit den medizinischen Injektionskanülen nicht die Gewebestrukturen unterhalb des Implantats perforieren beziehungsweise schädigen kann.

Das erfindungsgemäße Implantat hat bevorzugt die Gestalt einer leicht nach oben gewölbten Scheibe. Die untere Wandung und die obere Wandung sind bevorzugt aus einem weichen, gummielastischen Material geformt. Das Implantat kann sich dadurch an die Oberflächengestalt des Implantationsortes anpassen. Das Implantat ist bevorzugt flach und in einer Draufsicht besonders bevorzugt kreisförmig oder ellipsenförmig. Es hat vorzugsweise einen Durchmesser im Bereich von 3 mm bis maximal 20 mm. Größere Implantate sind besonders für die lokale Wirkstofffreisetzung an der Gelenkskapsel des Kniegelenks bestimmt. Bei Implantaten mit kleinem Durchmesser ist eine Implantation im Bereich der Fingergelenke und des Handgrundgelenks möglich. Die mindestens eine Durchbrechung hat bevorzugt einen Durchmesser von kleiner 0,5 mm und besonders bevorzugt kleiner von 0,25 mm. Es ist auch möglich, zwei oder mehrere Durchbrechungen in der unteren Wandung anzuordnen. Es wurde im Rahmen der vorliegenden Erfindung erkannt, dass es vorteilhaft ist, wenn das Implantat nur auf der dem zu behandelnden Gewebe zugewandten Seite pharmazeutische Wirkstoffe in Form von Wirkstofflösungen verzögert freisetzen kann.

Das Implantat wird bevorzugt in der Weise verwendet, dass zuerst das Implantat subkutan an die äußere Oberfläche der Gelenkkapsel des zu behandelnden Gelenks implantiert wird. Es kann ein bereits mit Wirkstofflösung befülltes Implantat aber auch ein noch nicht gefülltes Implantat implantiert werden. Nach erfolgter Positionierung des Implantats kann dieses an das umgebende Weichgewebe zur Fixierung angenäht werden. Wenn das Implantat im nicht mit Wirkstofflösung befüllten Zustand implantiert wurde kann der medizinische Anwender dieses mit beliebigen pharmazeutischen Wirkstofflösungen dotieren, besonders bevorzugt mit Lösungen von Immunmodulatoren dotieren. Dazu wird die zu applizierende Wirkstofflösung in eine Spritze aufgezogen, wobei die Spritze mit einer Injektionskanüle (Injektionsnadel) mit einem Durchmesser kleiner 0,5 mm verbunden ist. Danach wird durch die Haut des Patienten in Richtung des Implantats gestochen und die obere Wandung perforiert. Die Injektionskanüle wird dann durch den Durchstechschutz an einem weiteren Vordringen gestoppt. Dann wird die Wirkstofflösung in den Innenraum beziehungsweise in den Hohlraum appliziert. Nach Beendigung der Applikation wird die Injektionskanüle aus dem Innenraum beziehungsweise aus dem Hohlraum und aus der oberen Wandung herausgezogen. Die Perforation in der oberen Wandung verschließt sich auf Grund der Rückstellkraft des gummielastischen Materials der oberen Wandung. Die Freisetzung der Wirkstofflösung erfolgt durch Diffusion aus der mindestens einen für Flüssigkeiten durchlässigen Durchführung an der Unterseite der unteren Wandung. Selbstverständlich kann das Implantat auch vor der Implantation mit Hilfe der Spritze mit einem Wirkstoff beziehungsweise mit einer Flüssigkeit enthaltend zumindest einen Wirkstoff befüllt werden.

Als pharmazeutische Wirkstoffe kommen bevorzugt Immunmodulatoren in Betracht, die ausreichend über eine längere Zeit hydrolytisch stabil sind. Bevorzugte Immunmodulatoren sind Cyclosporin A (CAS 59865-13-3), Tacrolimus (CAS 104987-11-3) und Evrolimus (CAS 159351-69-6). Daneben können Kortikosteroide wie Cortison (CAS 53-06-5), Betamethason (CAS 378-44-9), Triamcinolon (CAS 124-94-7), Dexamethason (CAS 50-02-2), Dexamethasonphosphat (CAS 2392-39-4) als Immunmodulatoren eingesetzt werden. Des Weiteren könnte auch der Immunmodulator Apremilast (CAS 608141-41-9) eingesetzt werden. Besonders bevorzugt sind Immunmodulatoren, die eine sehr geringe Löslichkeit in Wasser bei Raumtemperatur haben.

Nach erfolgter Wirkstofffreisetzung kann das Implantat mehrfach mit Hilfe einer Spritze und einer Injektionskanüle mit Wirkstofflösung aufgefüllt werden, ohne dass eine Explantation des Implantats notwendig wäre.

Ein beispielhaftes erfindungsgemäßes Implantat zur lokalen Wirkstofffreisetzung ist zusammengesetzt aus
a) einem scheibenförmigen Grundkörper als untere Wandung mit zumindest einer Durchführung, welche die Oberseite des scheibenförmigen Grundkörpers mit der Unterseite des scheibenförmigen Grundkörpers flüssigkeitsdurchlässig verbindet,
b) einer nicht mit Injektionskanülen durchstechbaren Scheibe als Durchstechschutz, die auf dem scheibenförmigen Grundkörper aufgelagert ist,
c) einer flüssigkeitsundurchlässigen Abdeckung als obere Wandung, wobei die flüssigkeitsundurchlässige Abdeckung scheibenförmig und elastisch verformbar ist und mit dem scheibenförmigen Grundkörper verbunden ist, und
d) einem Innenraum, der von der Innenseite der flüssigkeitsundurchlässigen Abdeckung und der Oberseite der nicht mit Injektionskanülen durchstechbaren Scheibe begrenzt wird.

Erfindungsgemäß kann vorgesehen sein, dass die flüssigkeitsundurchlässige Abdeckung aus einem gummielastischen Kunststoff gebildet ist oder einen solchen in einem mittleren Bereich aufweist, wobei die flüssigkeitsundurchlässige Abdeckung mit Injektionskanülen durchstechbar ist, wobei die Durchstechöffnung nach Entfernung der Injektionskanüle durch die Rückstellkraft des gummielastischen Kunststoffs wieder verschließbar ist.

Die flüssigkeitsundurchlässige Abdeckung kann am gesamten umlaufenden flüssigkeitsundurchlässig mit dem scheibenförmigen Grundkörper verbunden sein. Dadurch kann die Wirkstofflösung nur aus der zumindest einen Durchführung des scheibenförmigen Grundkörpers austreten.

Vorteilhaft ist es, wenn an der Unterseite der flüssigkeitsundurchlässigen Abdeckung Stege und/oder Noppen als Abstandhalter angeordnet sind. Das Implantat wird durch Weichgewebe überlagert. Dieses kann Druck auf die flüssigkeitsundurchlässige Abdeckung ausüben, wobei die die flüssigkeitsundurchlässige Abdeckung dadurch Druck auf die Wirkstofflösung im Hohlraum ausüben kann. Eine Beschleunigung des Austritts der Wirkstofflösung durch die zumindest eine Durchführung in dem scheibenförmigen Grundkörper kann dadurch beschleunigt werden. Durch Stege und/oder Noppen an der Unterseite der flüssigkeitsundurchlässigen Abdeckung wird eine Bewegung der flüssigkeitsundurchlässigen Abdeckung in Richtung des scheibenförmigen Grundkörpers verhindert. Dadurch wird der Innenraum beziehungsweise der Hohlraum druckentlastet und ein Auspressen der Wirkstofflösung aus dem Hohlraum verhindert.

Im Folgenden werden neun weitere Ausführungsbeispiele der Erfindung und der Ablauf eines erfindungsgemäßen Verfahrens anhand von dreiunddreißig schematisch dargestellten Figuren erläutert, ohne jedoch dabei die Erfindung zu beschränken. Dabei zeigt:
Figur 1: eine schematische perspektivische Querschnittansicht durch ein erstes beispielhaftes erfindungsgemäßes Implantat zur lokalen Applikation von Flüssigkeiten;
Figur 2: eine schematische perspektivische Explosionsdarstellung des ersten Implantats nach Figur 1;
Figur 3: eine schematische Querschnittansicht des ersten Implantats nach den Figuren 1 und 2;
Figur 4: eine schematische perspektivische Teilschnittansicht auf das erste Implantat nach den Figuren 1 bis 3;
Figur 5: zwei schematische Querschnittansichten als Detailvergrößerungen des ersten Implantats nach den Figuren 1 bis 4;
Figur 6: eine schematische perspektivische Aufsicht auf die Unterseite eines zweiten beispielhaften Implantats zur lokalen Applikation von Flüssigkeiten;
Figur 7: eine schematische Querschnittansicht des zweiten Implantats nach Figur 6;
Figur 8: eine schematische perspektivische Aufsicht auf die Oberseite des zweiten beispielhaften Implantats nach den Figuren 6 und 7;
Figur 9: eine schematische perspektivische Querschnittansicht durch ein drittes beispielhaftes Implantat zur lokalen Applikation von Flüssigkeiten;
Figur 10: eine schematische perspektivische Aufsicht auf die Unterseite des dritten Implantats nach Figur 9;
Figur 11: eine schematische Querschnittansicht des dritten Implantats nach den Figuren 9 und 10;
Figur 12: eine schematische perspektivische Querschnittansicht durch ein viertes beispielhaftes Implantat zur lokalen Applikation von Flüssigkeiten;
Figur 13: eine schematische Querschnittansicht des vierten Implantats nach Figur 12;
Figur 14: eine schematische perspektivische Querschnittansicht durch ein fünftes beispielhaftes Implantat zur lokalen Applikation von Flüssigkeiten;
Figur 15: eine schematische Querschnittansicht des fünften Implantats nach Figur 14;
Figur 16: eine schematische perspektivische Querschnittansicht durch ein sechstes beispielhaftes Implantat zur lokalen Applikation von Flüssigkeiten;
Figur 17: eine schematische Querschnittansicht des sechsten Implantats nach Figur 16;
Figur 18: eine schematische perspektivische Aufsicht auf die Unterseite eines siebten beispielhaften Implantats zur lokalen Applikation von Flüssigkeiten;
Figur 19: eine schematische Querschnittansicht des siebten Implantats nach Figur 18;
Figur 20: eine schematische perspektivische Aufsicht auf einen Durchstechschutz, wie er in den beispielhaften Implantaten nach den Figuren 1 bis 19 enthalten ist;
Figur 21: eine schematische perspektivische Querschnittansicht durch ein achtes beispielhaftes Implantat zur lokalen Applikation von Flüssigkeiten;
Figur 22: eine schematische Querschnittansicht des achten Implantats nach Figur 21;
Figur 23: eine schematische perspektivische Aufsicht auf die Unterseite eines Durchstechschutzes des achten Implantats nach den Figuren 21 und 22;
Figur 24: eine schematische perspektivische Querschnittansicht durch ein neuntes beispielhaftes Implantat zur lokalen Applikation von Flüssigkeiten;
Figur 25: eine schematische Querschnittansicht des neunten Implantats nach Figur 24;
Figur 26: eine schematische perspektivische Aufsicht auf die Unterseite eines Durchstechschutzes des neunten Implantats nach den Figuren 24 und 25;
Figur 27: eine schematische Seitenansicht auf eine Spritze und das neunte Implantat vor dem Einstechen der Injektionskanüle in das neunte Implantat;
Figur 28: eine schematische Querschnittansicht durch das neunte Implantat und die in das neunte Implantat eingestochene Spritze kurz vor dem Befüllen des neunten Implantats mit einer Flüssigkeit aus der Spritze;
Figur 29: eine schematische Querschnittansicht durch das neunte Implantat und die in das neunte Implantat eingestochene Spritze während dem Befüllen des neunten Implantats mit der Flüssigkeit aus der Spritze;
Figur 30: eine schematische Querschnittansicht durch das neunte Implantat und die aus dem neunten Implantat wieder herausgezogene Spritze nach dem Befüllen des neunten Implantats;
Figur 31: eine schematische Querschnittansicht durch das neunte Implantat und die aus dem neunten Implantat wieder herausgezogene Spritze während des Austritts von Flüssigkeit aus dem neunten Implantat;
Figur 32: eine Detailansicht des neunten Implantats mit eingestochener Injektionskanüle als Ausschnittvergrößerung der Figur 28; und
Figur 33: eine Detailansicht des neunten Implantats als Ausschnittvergrößerung der Figur 31.

In den Figuren und in der folgenden Beschreibung zu den anhand der Figuren erläuterten neun Ausführungsbeispielen der vorliegenden Erfindung, das heißt, der neun erfindungsgemäßen Implantate, werden teilweise für unterschiedliche Ausführungsbeispiele beziehungsweise für unterschiedliche Implantate und für unterschiedliche Einzelteile der Implantate die gleichen Bezugszeichen bei gleichen oder gleichartigen Teilen verwendet, um die Vergleichbarkeit der Ausführungsbeispiele und die Lesbarkeit zu vereinfachen.

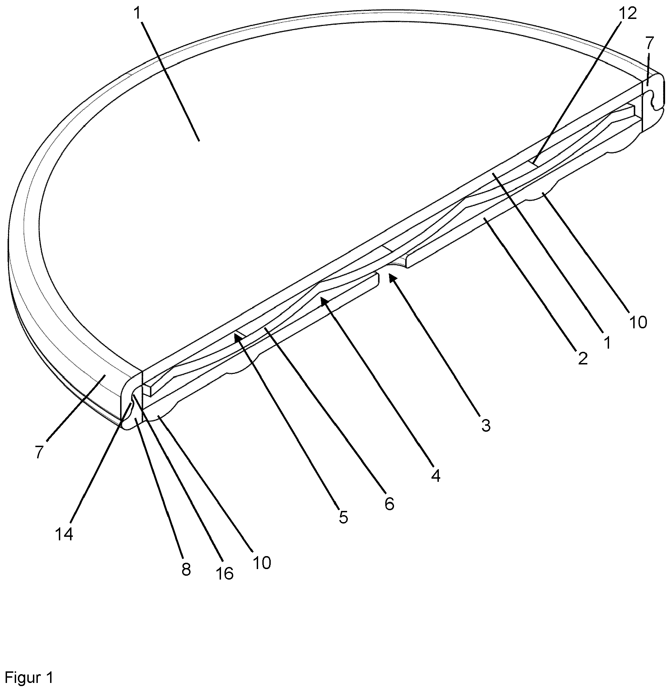

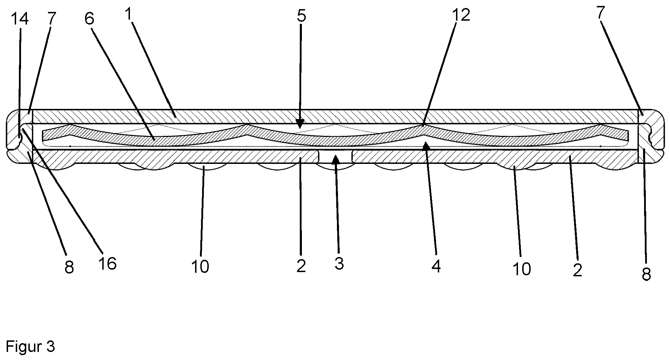

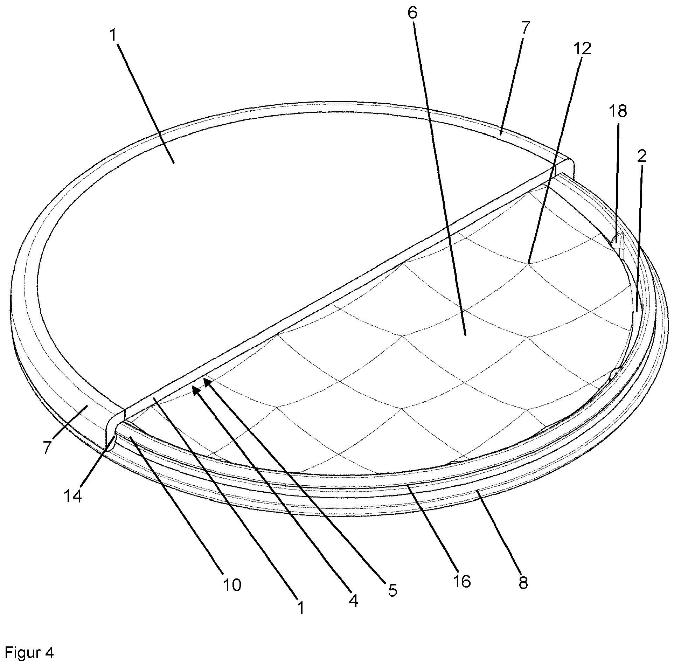

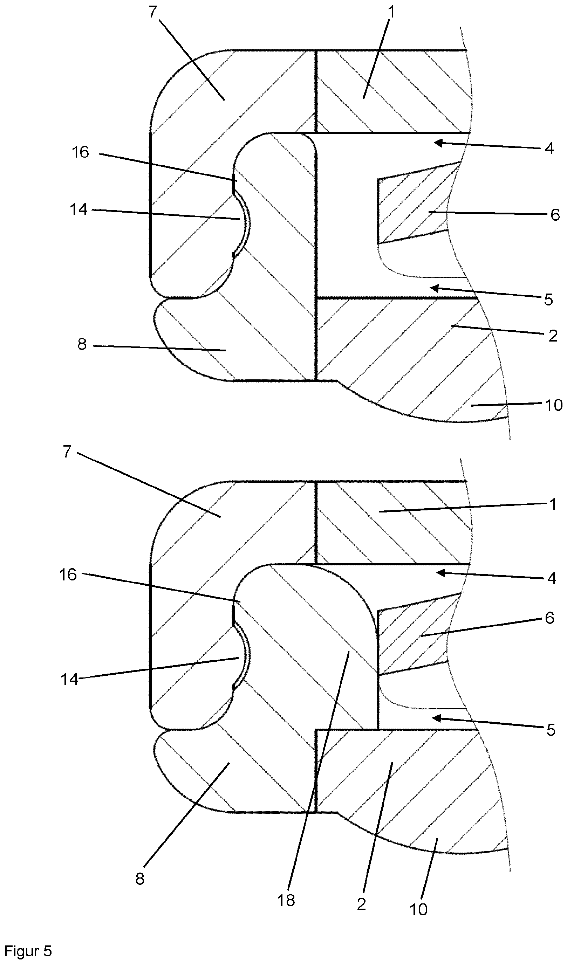

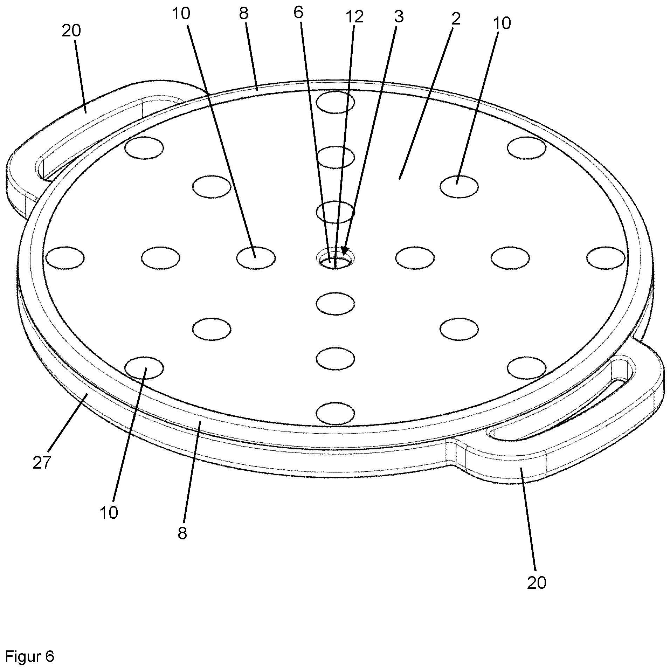

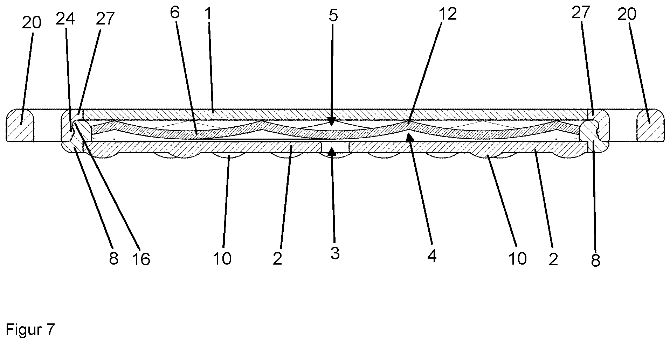

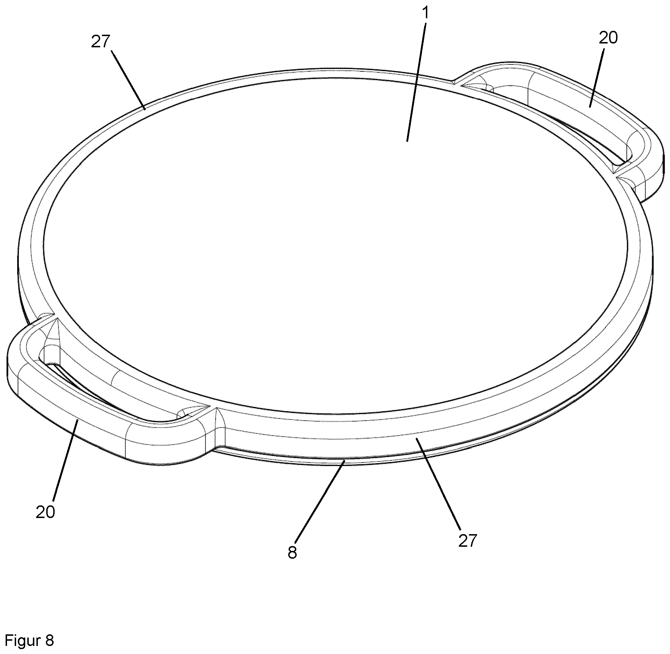

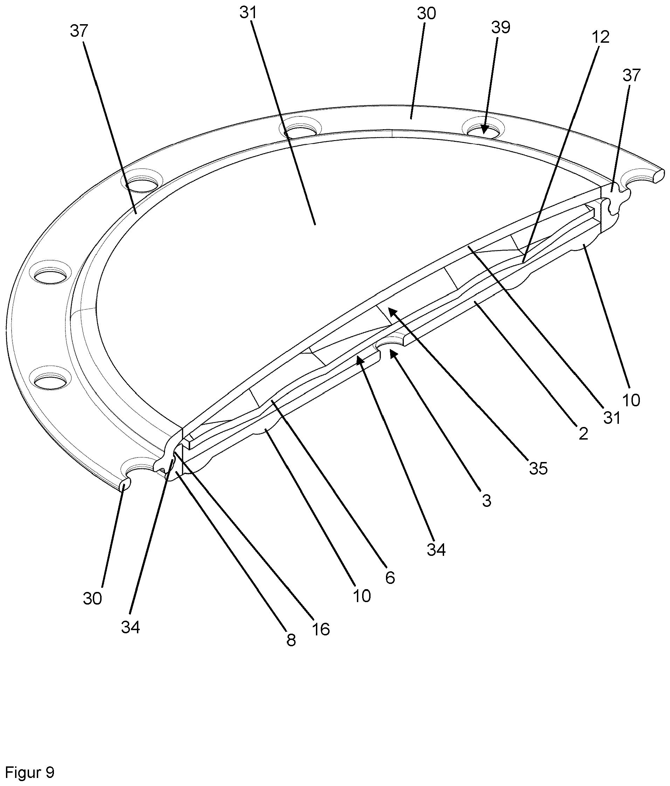

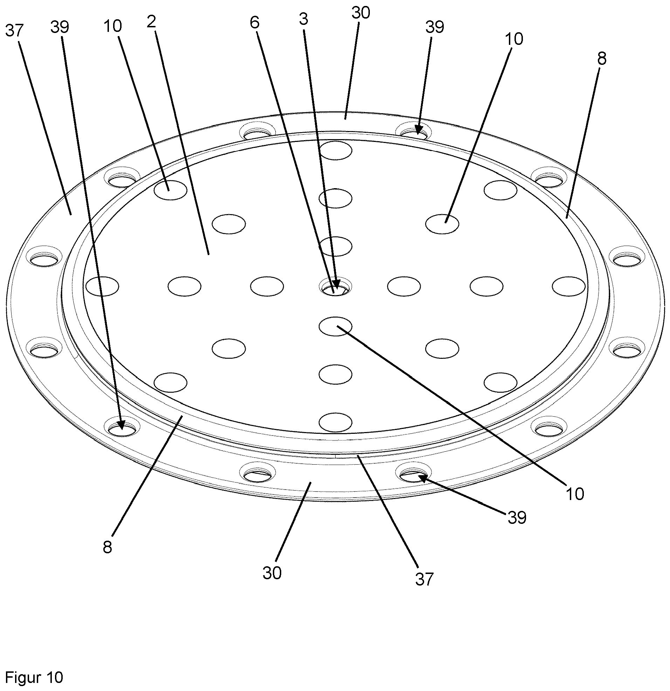

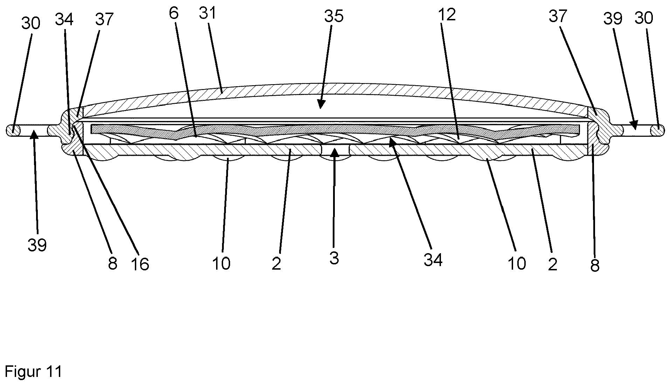

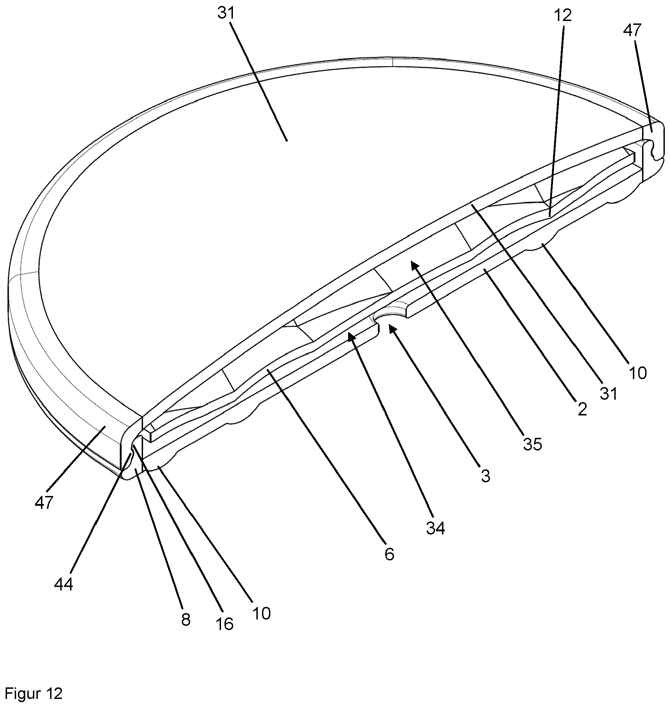

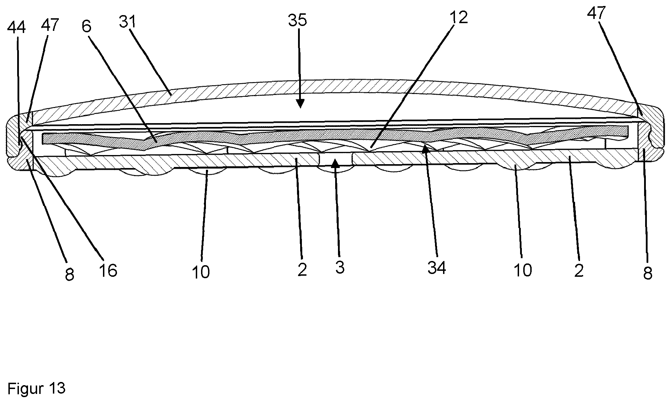

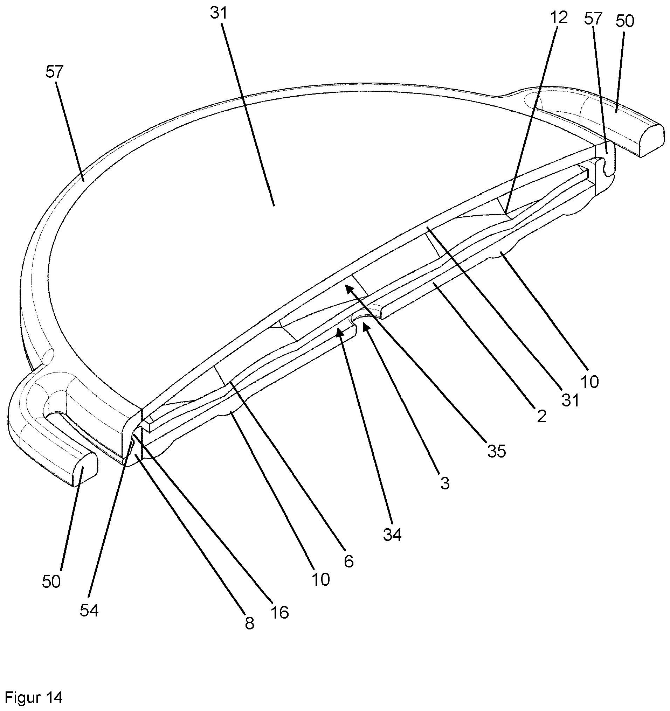

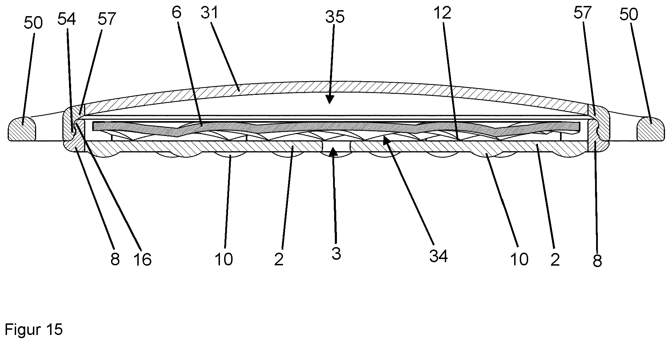

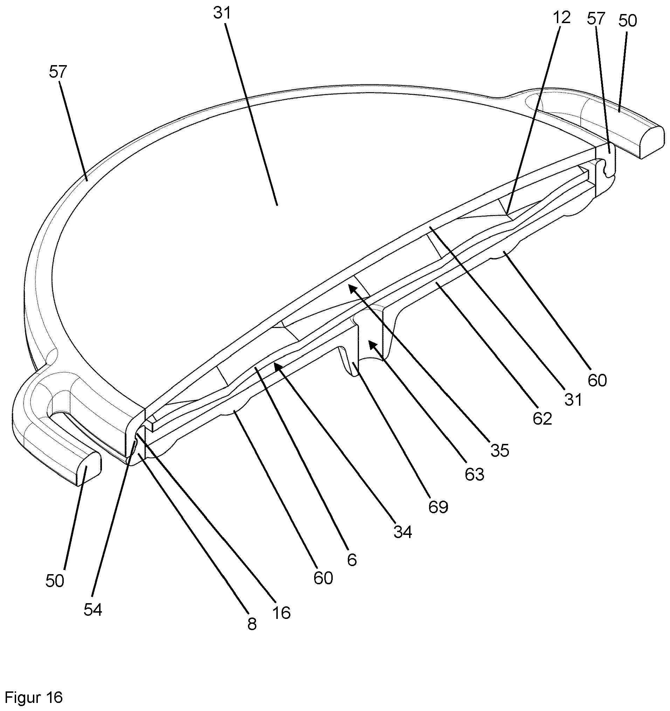

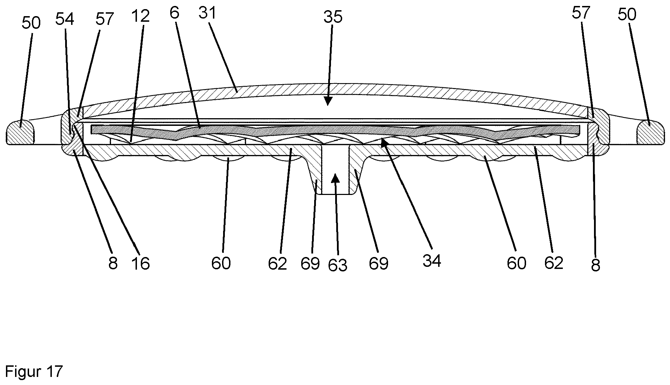

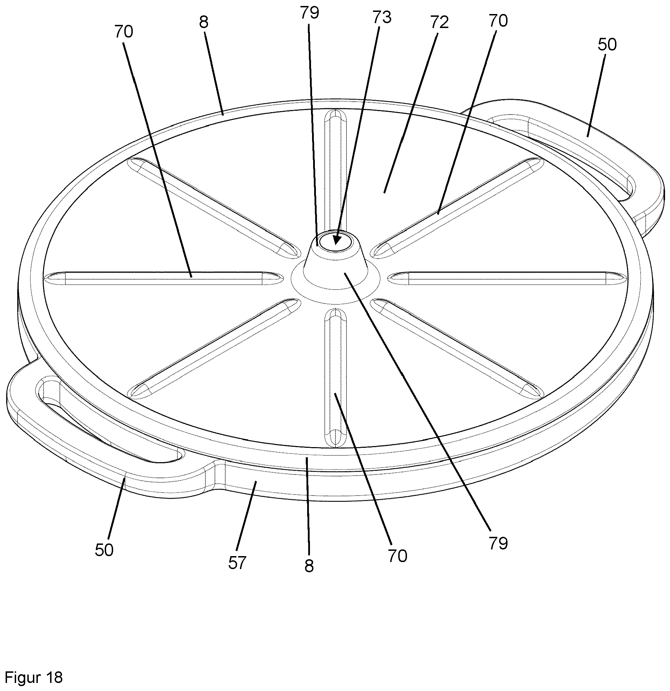

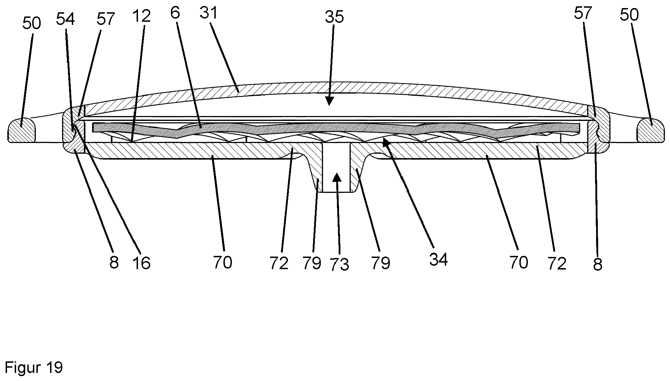

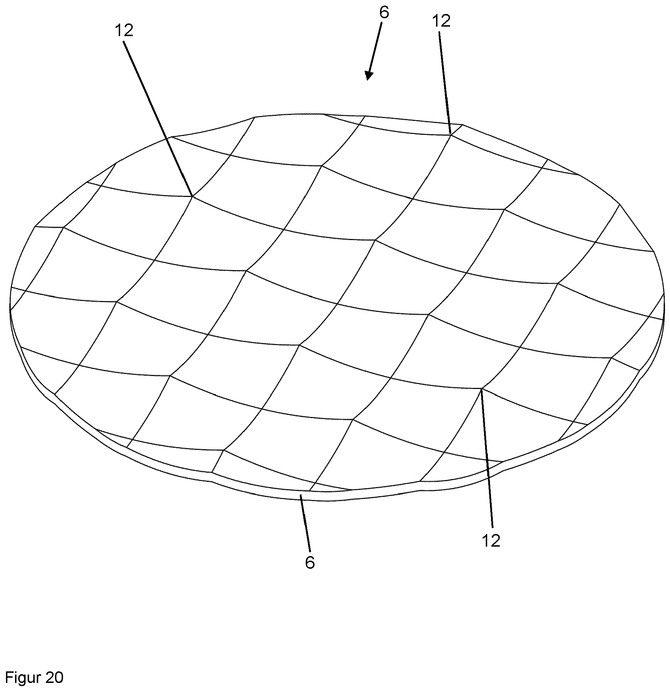

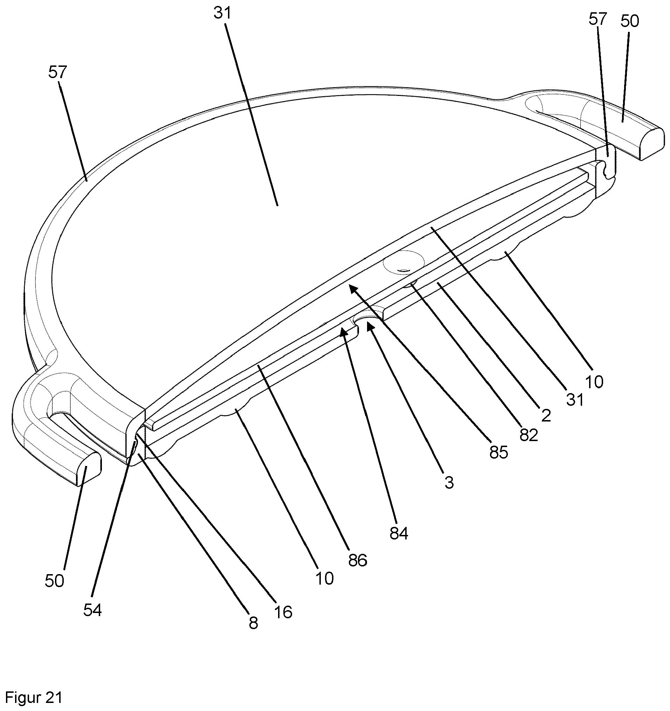

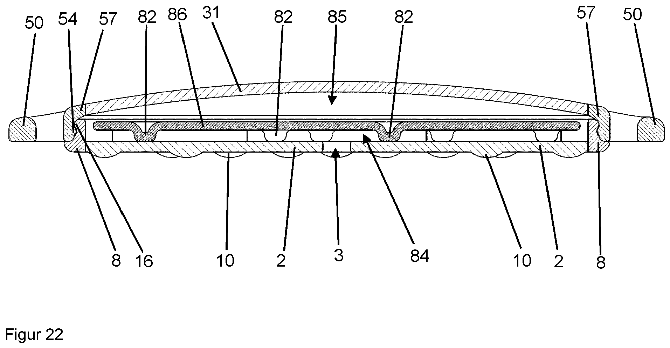

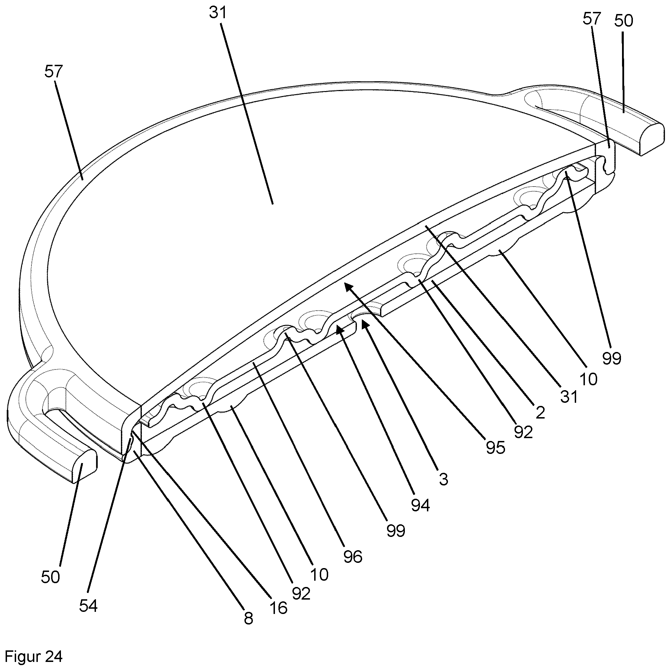

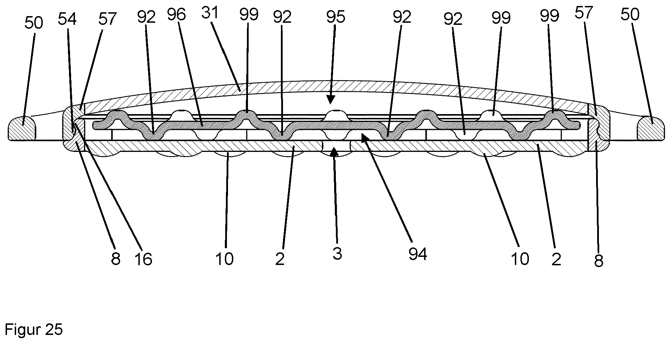

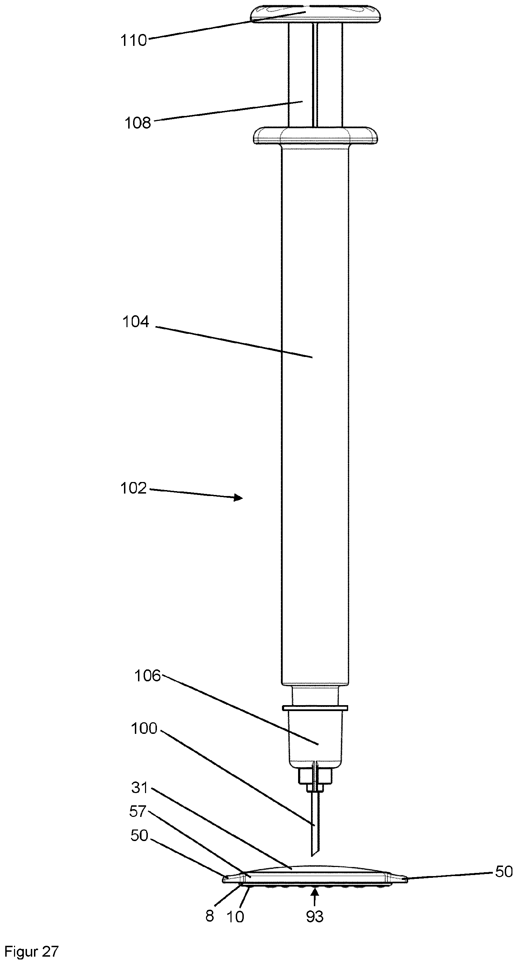

Die Figuren 1 bis 5 zeigen ein erstes beispielhaftes erfindungsgemäßes Implantat zur lokalen Applikation einer Flüssigkeit in unterschiedlichen Darstellungen. Die Figuren 6 bis 8 zeigen ein zweites beispielhaftes erfindungsgemäßes Implantat zur lokalen Applikation einer Flüssigkeit in unterschiedlichen Darstellungen. Die Figuren 9 bis 11 zeigen ein drittes, die Figuren 12 und 13 ein viertes, die Figuren 14 und 15 ein fünftes, die Figuren 16 und 17 ein sechstes und die Figuren 18 und 19 ein siebtes beispielhaftes erfindungsgemäßes Implantat zur lokalen Applikation einer Flüssigkeit in unterschiedlichen Darstellungen. Die Figur 20 zeigt einen Durchstechschutz, wie in den ersten sieben Ausführungsbeispielen zum Einsatz kommt. Die Figuren 21 bis 23 zeigen ein achtes beispielhaftes erfindungsgemäßes Implantat zur lokalen Applikation einer Flüssigkeit in unterschiedlichen Darstellungen mit einem anderen Durchstechschutz. Die Figuren 24 bis 26 zeigen ein neuntes beispielhaftes erfindungsgemäßes Implantat zur lokalen Applikation einer Flüssigkeit in unterschiedlichen Darstellungen. In den Figuren 27 bis 33 ist der Ablauf eines erfindungsgemäßen Verfahrens zum Befüllen eines Implantats mit einer Spritze anhand des neunten beispielhaften Implantats gezeigt, wobei das Verfahren ohne weiteres auch auf die anderen Ausführungsbeispiele übertragbar ist.

Das Implantat nach dem ersten Ausführungsbeispiel nach den Figuren 1 bis 5 weist eine obere Wandung 1 auf. Die obere Wandung 1 ist mit einer üblichen medizinischen Injektionskanüle mit manueller Kraft durchstechbar (so wie das für das neunte Ausführungsbeispiel in den Figuren 27 bis 33 gezeigt ist). Die obere Wandung 1 kann eine Oberseite des Implantats bilden. Das Material für die obere Wandung 1 kann so beschaffen sein, dass die obere Wandung 1 sich nach dem Herausziehen einer durchgestochenen Injektionskanüle wieder selbsttätig verschließt. Hierzu kann die obere Wandung aus einem gummielastischen Kunststoff oder aus Gummi bestehen beziehungsweise zumindest in einem zentralen Bereich einen gummielastischen Kunststoff oder Gummi aufweisen. Derartige selbstdichtende Membranen kommen beispielsweise bei Fläschchen zum Aufziehen von Spritzen zum Einsatz.

Auf der der Oberseite gegenüberliegenden Unterseite des Implantats ist eine untere Wandung 2 mit einer Durchführung 3 angeordnet, wobei auch mehrere Durchführungen (nicht gezeigt) vorgesehen sein können. Die Durchführung 3 kann zentral in der Mitte der unteren Wandung 2 angeordnet sein. Die Durchführung 3 verbindet die Oberseite der unteren Wandung 2 mit der Unterseite der oberen Wandung 2 und damit mit der Unterseite des Implantats.

Die obere Wandung 1 und die untere Wandung 2 begrenzen einen Hohlraum 4 im Inneren des Implantats. Die Durchführung 3 verbindet den Hohlraum 4 des Implantats mit der Umgebung des Implantats flüssigkeitsdurchlässig. Ein Innenraum 5, der ein Teilstück des Hohlraums 4 ist, kann zwischen einem Durchstechschutz 6 im Hohlraum 4 und der oberen Wandung 1 angeordnet sein. Der Durchstechschutz 6 ist scheibenförmig und ist im Hohlraum 4 zwischen der unteren Wandung 2 und der oberen Wandung 1 angeordnet. Der Durchstechschutz 6 kann scheibenförmig sein. Der Durchstechschutz 6 kann einen kleineren Durchmesser aufweisen als der Hohlraum 4, so dass er den Hohlraum 4 nicht flüssigkeitsdicht in zwei Teile trennt, beziehungsweise in zwei, miteinander flüssigkeitsdurchlässig verbundene Bereiche trennt. Der Durchstechschutz 6 ist im Detail in Figur 20 gezeigt. Der Durchstechschutz 6 ist nicht mit einer üblichen medizinischen Injektionskanüle mit manueller Kraft durchstechbar (so wie das für das neunte Ausführungsbeispiel in den Figuren 27 bis 33 gezeigt ist).

Die obere Wandung 1 kann einen umlaufenden oberen Rand 7 aufweisen. Der obere Rand 7 der oberen Wandung 1 kann einen Kreis bilden, der die obere Wandung 1 umschließt. Die untere Wandung 2 kann einen umlaufenden unteren Rand 8 aufweisen. Der untere Rand 8 der unteren Wandung 2 kann einen Kreis bilden, der die untere Wandung 2 umschließt.

Die untere Wandung 2 kann an einer Unterseite Noppen 10 aufweisen, die von der Unterseite der unteren Wandung 2 abstehen. Auf diese Weise kann sichergestellt werden, dass das Implantat, wenn es mit der Unterseite der unteren Wandung 2 auf einer Unterlage aufliegt, dort einen Abstand einhält. Durch den so entstehenden Zwischenraum kann sich dann die Flüssigkeit, die durch die Durchführung 3 aus dem Hohlraum 4 austritt, entlang der Oberfläche der unteren Wandung 2 verteilen.

Der Durchstechschutz 6 kann eine wellenförmige Form aufweisen, die an der Oberseite und an seiner Unterseite des Durchstechschutzes 6 abstehende Strukturierungen 12 bildet (siehe Figur 20). Hiermit kann erreicht werden, dass eine Unterseite der oberen Wandung 1 von einer Oberseite des Durchstechschutzes 6 derart beabstandet ist, dass die Spitze einer Injektionskanüle in den Innenraum 5 eindringen kann, um dort die Flüssigkeit zu injizieren. Zudem kann so eine Oberseite der unteren Wandung 2 von der Unterseite des Durchstechschutzes 6 beabstandet sein, so dass eine Flüssigkeit sich problemlos in dem unteren Teil des Innenraums 4 ausbreiten kann und bis zu der Durchführung 3 ausbreiten kann.

Die obere Wandung 1 und die untere Wandung 2 können über den oberen Rand 7 und den unteren Rand 8 miteinander flüssigkeitsdicht verbunden sein, so dass der Hohlraum 4 bis auf die Durchführung 3 flüssigkeitsdicht verschlossen ist. Hierzu kann an dem oberen Rand 7 ein Rastring 14 ausgebildet sein und an dem unteren Rand 8 ein Rastring 16. Der Rastring 14 des oberen Rands 7 kann in den Rastring 16 des unteren Rands 8 greifen, wenn die obere Wandung 1 vollständig auf die untere Wandung 2 aufgesteckt wird.

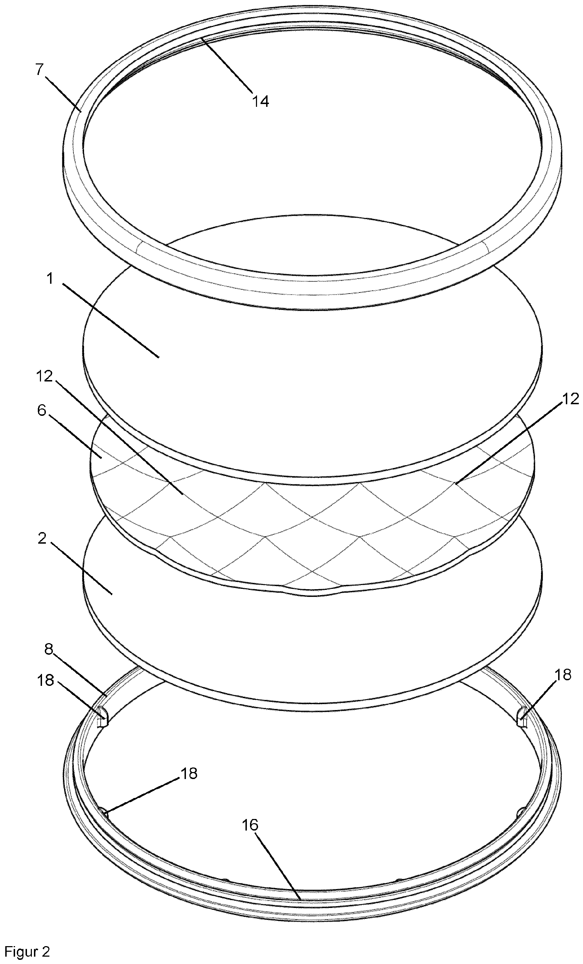

Die Verwendung eines solchen Implantats und damit ein erfindungsgemäßes Verfahren können dadurch erfolgen, dass eine Injektionskanüle einer Spritze in einem mittleren Bereich durch die obere Wandung 1 gestochen wird. Anschließend kann eine Flüssigkeit aus der Spritze in den Innenraum 5 injiziert werden. Der Durchstechschutz 6 kann mit Hilfe von in den Hohlraum 4 ragenden Nasen 18 zentral gehalten werden, so dass der Durchstechschutz 6 seitlich von dem unteren Rand 8 beabstandet ist (siehe Figuren 2 und 4 und Figur 5 unten). Die Nasen 18 können allseitig an einem inneren Umfang des unteren Rands 8 angeordnet sein. Die Flüssigkeit kann so von dem Innenraum 5 in den unteren Teil des Hohlraums 4 zwischen dem Durchstechschutz 6 und der unteren Wandung 2 fließen.

Die untere Wandung 2 und die obere Wandung 1 können elastisch expandierbar sein. Durch das Einspritzen der Flüssigkeit in den Hohlraum 4 kann der Hohlraum 4 dann elastisch verformt, beziehungsweise elastisch expandiert werden. Die Flüssigkeit in dem Hohlraum 4 steht dann unter einem elastischen Druck. Dadurch kann die Flüssigkeit durch die Durchführung 3 aus dem Hohlraum 4 ausgepresst werden. Das Implantat kann zuvor oder auch danach subkutan im Bereich eines Gelenks oder an einer anderen zu behandelnden Stelle Implantiert werden. Die Flüssigkeit kann sich nach dem Austreten aus dem Hohlraum 4 durch die Durchführung 3 entlang des Zwischenraums zwischen der Unterseite der unteren Wandung 2 und der Auflage ausbreiten und verteilen. Wenn keine Flüssigkeit mehr aus dem Hohlraum 4 herausfließt, kann entweder ein Druck auf das Implantat ausgeübt werden, um einen Gegendruck zu überwinden und weitere Flüssigkeit aus dem Implantat freizusetzen oder es kann neue Flüssigkeit mit einer Spritze in den Innenraum 5 injiziert werden.

Ein erfindungsgemäßes Verfahren ist im Detail zu den Figuren 24 bis 33 im Zusammenhang mit dem neunten beispielhaften Implantat weiter unten beschrieben. Das Verfahren lässt sich aber ohne weiteres auch auf das eben beschriebene Implantat übertragen.

Das Implantat nach dem zweiten Ausführungsbeispiel nach den Figuren 6 bis 8 unterscheidet sich von dem ersten Ausführungsbeispiel durch zwei Laschen 20 an einem oberen umlaufenden Rand 27 einer oberen Wandung 1 des Implantats, mit denen das Implantat an Weichgewebe angenäht werden kann. Ansonsten gleicht das zweite Implantat dem ersten Implantat nach den Figuren 1 bis 5. Die obere Wandung 1 ist mit einer üblichen medizinischen Injektionskanüle mit manueller Kraft durchstechbar (so wie das für das neunte Ausführungsbeispiel in den Figuren 27 bis 33 gezeigt ist). Die obere Wandung 1 kann eine Oberseite des Implantats bilden. Das Material für die obere Wandung 1 kann so beschaffen sein, dass die obere Wandung 1 sich nach dem Herausziehen einer durchgestochenen Injektionskanüle wieder selbsttätig verschließt. Hierzu kann die obere Wandung aus einem gummielastischen Kunststoff oder aus Gummi bestehen beziehungsweise zumindest in einem zentralen Bereich einen gummielastischen Kunststoff oder Gummi aufweisen. Derartige selbstdichtende Membranen kommen beispielsweise bei Fläschchen zum Aufziehen von Spritzen zum Einsatz.

Auf der der Oberseite gegenüberliegenden Unterseite des Implantats ist eine untere Wandung 2 mit einer Durchführung 3 angeordnet, wobei auch mehrere Durchführungen (nicht gezeigt) vorgesehen sein können. Die Durchführung 3 kann zentral in der Mitte der unteren Wandung 2 angeordnet sein. Die Durchführung 3 verbindet die Oberseite der unteren Wandung 2 mit der Unterseite der oberen Wandung 2 und damit mit der Unterseite des Implantats.

Die obere Wandung 1 und die untere Wandung 2 begrenzen einen Hohlraum 4 im Inneren des Implantats. Die Durchführung 3 verbindet den Hohlraum 4 des Implantats mit der Umgebung des Implantats flüssigkeitsdurchlässig. Ein Innenraum 5, der ein Teilstück des Hohlraums 4 ist, kann zwischen einem Durchstechschutz 6 im Hohlraum 4 und der oberen Wandung 1 angeordnet sein. Der Durchstechschutz 6 ist scheibenförmig und ist im Hohlraum 4 zwischen der unteren Wandung 2 und der oberen Wandung 1 angeordnet. Der Durchstechschutz 6 kann scheibenförmig sein. Der Durchstechschutz 6 kann einen kleineren Durchmesser aufweisen als der Hohlraum 4, so dass er den Hohlraum 4 nicht flüssigkeitsdicht in zwei Teile trennt, beziehungsweise in zwei, miteinander flüssigkeitsdurchlässig verbundene Bereiche trennt. Der Durchstechschutz 6 ist im Detail in Figur 20 gezeigt. Der Durchstechschutz 6 ist nicht mit einer üblichen medizinischen Injektionskanüle mit manueller Kraft durchstechbar (so wie das für das neunte Ausführungsbeispiel in den Figuren 27 bis 33 gezeigt ist).

Der obere Rand 27 der oberen Wandung 1 kann einen Kreis bilden, der die obere Wandung 1 umschließt. Die untere Wandung 2 kann einen umlaufenden unteren Rand 8 aufweisen. Der untere Rand 8 der unteren Wandung 2 kann einen Kreis bilden, der die untere Wandung 2 umschließt.

Die untere Wandung 2 kann an einer Unterseite Noppen 10 aufweisen, die von der Unterseite der unteren Wandung 2 abstehen. Auf diese Weise kann sichergestellt werden, dass das Implantat, wenn es mit der Unterseite der unteren Wandung 2 auf einer Unterlage aufliegt, dort einen Abstand einhält. Durch den so entstehenden Zwischenraum kann sich dann die Flüssigkeit, die durch die Durchführung 3 aus dem Hohlraum 4 austritt, entlang der Oberfläche der unteren Wandung 2 verteilen.

Der Durchstechschutz 6 kann eine wellenförmige Form aufweisen, die an der Oberseite und an seiner Unterseite des Durchstechschutzes 6 abstehende Strukturierungen 12 bildet (siehe Figur 20). Hiermit kann erreicht werden, dass eine Unterseite der oberen Wandung 1 von einer Oberseite des Durchstechschutzes 6 derart beabstandet ist, dass die Spitze einer Injektionskanüle in den Innenraum 5 eindringen kann, um dort die Flüssigkeit zu injizieren. Zudem kann so eine Oberseite der unteren Wandung 2 von der Unterseite des Durchstechschutzes 6 beabstandet sein, so dass eine Flüssigkeit sich problemlos in dem unteren Teil des Innenraums 4 ausbreiten kann und bis zu der Durchführung 3 ausbreiten kann.

Die obere Wandung 1 und die untere Wandung 2 können über den oberen Rand 27 und den unteren Rand 8 miteinander flüssigkeitsdicht verbunden sein, so dass der Hohlraum 4 bis auf die Durchführung 3 flüssigkeitsdicht verschlossen ist. Hierzu kann an dem oberen Rand 27 ein Rastring 24 ausgebildet sein und an dem unteren Rand 8 ein Rastring 16. Der Rastring 24 des oberen Rands 27 kann in den Rastring 16 des unteren Rands 8 greifen, wenn die obere Wandung 1 vollständig auf die untere Wandung 2 aufgesteckt wird.

Die Verwendung eines solchen Implantats und damit ein erfindungsgemäßes Verfahren können dadurch erfolgen, dass eine Injektionskanüle einer Spritze in einem mittleren Bereich durch die obere Wandung 1 gestochen wird. Anschließend kann eine Flüssigkeit aus der Spritze in den Innenraum 5 injiziert werden. Der Durchstechschutz 6 kann mit Hilfe von in den Hohlraum 4 ragenden Nasen (nicht zu sehen, aber analog den Nasen 18 nach dem ersten Ausführungsbeispiel) zentral gehalten werden, so dass der Durchstechschutz 6 seitlich von dem unteren Rand 8 beabstandet ist (analog den Figuren 2 und 4 und Figur 5 unten). Die Nasen können allseitig an einem inneren Umfang des unteren Rands 8 angeordnet sein. Die Flüssigkeit kann so von dem Innenraum 5 in den unteren Teil des Hohlraums 4 zwischen dem Durchstechschutz 6 und der unteren Wandung 2 fließen.

Die untere Wandung 2 und die obere Wandung 1 können elastisch expandierbar sein. Durch das Einspritzen der Flüssigkeit in den Hohlraum 4 kann der Hohlraum 4 dann elastisch verformt, beziehungsweise elastisch expandiert werden. Die Flüssigkeit in dem Hohlraum 4 steht dann unter einem elastischen Druck. Dadurch kann die Flüssigkeit durch die Durchführung 3 aus dem Hohlraum 4 ausgepresst werden. Das Implantat kann zuvor oder auch danach subkutan im Bereich eines Gelenks oder an einer anderen zu behandelnden Stelle Implantiert werden und mit Hilfe der Laschen 20 an Weichgewebe angenäht und fixiert werden. Die Flüssigkeit kann sich nach dem Austreten aus dem Hohlraum 4 durch die Durchführung 3 entlang des Zwischenraums zwischen der Unterseite der unteren Wandung 2 und der Auflage ausbreiten und verteilen. Wenn keine Flüssigkeit mehr aus dem Hohlraum 4 herausfließt, kann entweder ein Druck auf das Implantat ausgeübt werden, um einen Gegendruck zu überwinden und weitere Flüssigkeit aus dem Implantat freizusetzen oder es kann neue Flüssigkeit mit einer Spritze in den Innenraum 5 injiziert werden.

Ein erfindungsgemäßes Verfahren ist im Detail zu den Figuren 24 bis 33 im Zusammenhang mit dem neunten beispielhaften Implantat weiter unten beschrieben. Das Verfahren lässt sich aber ohne weiteres auch auf das eben beschriebene Implantat übertragen.

Das Implantat nach dem dritten Ausführungsbeispiel nach den Figuren 9 bis 11 unterscheidet sich von dem ersten Ausführungsbeispiel durch eine gewölbte obere Wandung 31 und einen anders geformten oberen Rand 37, in dem eine Vielzahl von Löchern 39 beziehungsweise Laschen 39 angeordnet sind, mit denen das Implantat an Weichgewebe angenäht werden kann. Ansonsten gleicht das dritte Implantat dem ersten Implantat nach den Figuren 1 bis 5. Die obere Wandung 31 ist mit einer üblichen medizinischen Injektionskanüle mit manueller Kraft durchstechbar (so wie das für das neunte Ausführungsbeispiel in den Figuren 27 bis 33 gezeigt ist). Die obere Wandung 31 kann eine Oberseite des Implantats bilden. Das Material für die obere Wandung 31 kann so beschaffen sein, dass die obere Wandung 31 sich nach dem Herausziehen einer durchgestochenen Injektionskanüle wieder selbsttätig verschließt. Hierzu kann die obere Wandung aus einem gummielastischen Kunststoff oder aus Gummi bestehen beziehungsweise zumindest in einem zentralen Bereich einen gummielastischen Kunststoff oder Gummi aufweisen. Derartige selbstdichtende Membranen kommen beispielsweise bei Fläschchen zum Aufziehen von Spritzen zum Einsatz.

Auf der der Oberseite gegenüberliegenden Unterseite des Implantats ist eine untere Wandung 2 mit einer Durchführung 3 angeordnet, wobei auch mehrere Durchführungen (nicht gezeigt) vorgesehen sein können. Die Durchführung 3 kann zentral in der Mitte der unteren Wandung 2 angeordnet sein. Die Durchführung 3 verbindet die Oberseite der unteren Wandung 2 mit der Unterseite der oberen Wandung 2 und damit mit der Unterseite des Implantats.

Die obere Wandung 31 und die untere Wandung 2 begrenzen einen Hohlraum 34 im Inneren des Implantats. Die Durchführung 3 verbindet den Hohlraum 34 des Implantats mit der Umgebung des Implantats flüssigkeitsdurchlässig. Ein Innenraum 35, der ein Teilstück des Hohlraums 34 ist, kann zwischen einem Durchstechschutz 6 im Hohlraum 34 und der oberen Wandung 31 angeordnet sein. Der Durchstechschutz 6 ist scheibenförmig und ist im Hohlraum 34 zwischen der unteren Wandung 2 und der oberen Wandung 31 angeordnet. Der Durchstechschutz 6 kann scheibenförmig sein. Der Durchstechschutz 6 kann einen kleineren Durchmesser aufweisen als der Hohlraum 34, so dass er den Hohlraum 34 nicht flüssigkeitsdicht in zwei Teile trennt, beziehungsweise in zwei, miteinander flüssigkeitsdurchlässig verbundene Bereiche trennt. Der Durchstechschutz 6 ist im Detail in Figur 20 gezeigt. Der Durchstechschutz 6 ist nicht mit einer medizinischen Injektionskanüle mit manueller Kraft durchstechbar (so wie das für das neunte Ausführungsbeispiel in den Figuren 27 bis 33 gezeigt ist).

Der obere Rand 37 der oberen Wandung 31 kann einen Kreis bilden, der die obere Wandung 31 umschließt. Die untere Wandung 2 kann einen umlaufenden unteren Rand 8 aufweisen. Der untere Rand 8 der unteren Wandung 2 kann einen Kreis bilden, der die untere Wandung 2 umschließt.

Die untere Wandung 2 kann an einer Unterseite Noppen 10 aufweisen, die von der Unterseite der unteren Wandung 2 abstehen. Auf diese Weise kann sichergestellt werden, dass das Implantat, wenn es mit der Unterseite der unteren Wandung 2 auf einer Unterlage aufliegt, dort einen Abstand einhält. Durch den so entstehenden Zwischenraum kann sich dann die Flüssigkeit, die durch die Durchführung 3 aus dem Hohlraum 34 austritt, entlang der Oberfläche der unteren Wandung 2 verteilen.

Der Durchstechschutz 6 kann eine wellenförmige Form aufweisen, die an der Oberseite und an seiner Unterseite des Durchstechschutzes 6 abstehende Strukturierungen 12 bildet (siehe Figur 20). Hiermit kann erreicht werden, dass eine Unterseite der oberen Wandung 31 von einer Oberseite des Durchstechschutzes 6 derart beabstandet ist, dass die Spitze einer Injektionskanüle in den Innenraum 35 eindringen kann, um dort die Flüssigkeit zu injizieren. Zudem kann so eine Oberseite der unteren Wandung 2 von der Unterseite des Durchstechschutzes 6 beabstandet sein, so dass eine Flüssigkeit sich problemlos in dem unteren Teil des Innenraums 34 ausbreiten kann und bis zu der Durchführung 3 ausbreiten kann.

Die obere Wandung 31 und die untere Wandung 2 können über den oberen Rand 37 und den unteren Rand 8 miteinander flüssigkeitsdicht verbunden sein, so dass der Hohlraum 34 bis auf die Durchführung 3 flüssigkeitsdicht verschlossen ist. Hierzu kann an dem oberen Rand 37 ein Rastring 34 ausgebildet sein und an dem unteren Rand 8 ein Rastring 16. Der Rastring 34 des oberen Rands 37 kann in den Rastring 16 des unteren Rands 8 greifen, wenn die obere Wandung 31 vollständig auf die untere Wandung 2 aufgesteckt wird.

Die Verwendung eines solchen Implantats und damit ein erfindungsgemäßes Verfahren können dadurch erfolgen, dass eine Injektionskanüle einer Spritze in einem mittleren Bereich durch die obere Wandung 31 gestochen wird. Anschließend kann eine Flüssigkeit aus der Spritze in den Innenraum 35 injiziert werden. Der Durchstechschutz 6 kann mit Hilfe von in den Hohlraum 34 ragenden Nasen (nicht zu sehen, aber analog den Nasen 18 nach dem ersten Ausführungsbeispiel) zentral gehalten werden, so dass der Durchstechschutz 6 seitlich von dem unteren Rand 8 beabstandet ist (analog den Figuren 2 und 4 und Figur 5 unten). Die Nasen können allseitig an einem inneren Umfang des unteren Rands 8 angeordnet sein. Die Flüssigkeit kann so von dem Innenraum 35 in den unteren Teil des Hohlraums 34 zwischen dem Durchstechschutz 6 und der unteren Wandung 2 fließen.

Die untere Wandung 2 und die obere Wandung 31 können elastisch expandierbar sein. Durch das Einspritzen der Flüssigkeit in den Hohlraum 34 kann der Hohlraum 34 dann elastisch verformt, beziehungsweise elastisch expandiert werden. Die Flüssigkeit in dem Hohlraum 34 steht dann unter einem elastischen Druck. Dadurch kann die Flüssigkeit durch die Durchführung 3 aus dem Hohlraum 34 ausgepresst werden. Das Implantat kann zuvor oder auch danach subkutan im Bereich eines Gelenks oder an einer anderen zu behandelnden Stelle Implantiert werden und mit Hilfe der Laschen 39 an Weichgewebe angenäht und fixiert werden. Die Flüssigkeit kann sich nach dem Austreten aus dem Hohlraum 34 durch die Durchführung 3 entlang des Zwischenraums zwischen der Unterseite der unteren Wandung 2 und der Auflage ausbreiten und verteilen. Wenn keine Flüssigkeit mehr aus dem Hohlraum 34 herausfließt, kann entweder ein Druck auf das Implantat ausgeübt werden, um einen Gegendruck zu überwinden und weitere Flüssigkeit aus dem Implantat freizusetzen oder es kann neue Flüssigkeit mit einer Spritze in den Innenraum 35 injiziert werden.

Ein erfindungsgemäßes Verfahren ist im Detail zu den Figuren 24 bis 33 im Zusammenhang mit dem neunten beispielhaften Implantat weiter unten beschrieben. Das Verfahren lässt sich aber ohne weiteres auch auf das eben beschriebene Implantat übertragen.

Das Implantat nach dem vierten Ausführungsbeispiel nach den Figuren 12 und 13 unterscheidet sich von dem ersten Ausführungsbeispiel durch eine gewölbte obere Wandung 31. Ansonsten gleicht das vierte Implantat dem ersten Implantat nach den Figuren 1 bis 5. Die obere Wandung 31 ist mit einer üblichen medizinischen Injektionskanüle mit manueller Kraft durchstechbar (so wie das für das neunte Ausführungsbeispiel in den Figuren 27 bis 33 gezeigt ist). Die obere Wandung 31 kann eine Oberseite des Implantats bilden. Das Material für die obere Wandung 31 kann so beschaffen sein, dass die obere Wandung 31 sich nach dem Herausziehen einer durchgestochenen Injektionskanüle wieder selbsttätig verschließt. Hierzu kann die obere Wandung aus einem gummielastischen Kunststoff oder aus Gummi bestehen beziehungsweise zumindest in einem zentralen Bereich einen gummielastischen Kunststoff oder Gummi aufweisen. Derartige selbstdichtende Membranen kommen beispielsweise bei Fläschchen zum Aufziehen von Spritzen zum Einsatz.

Auf der der Oberseite gegenüberliegenden Unterseite des Implantats ist eine untere Wandung 2 mit einer Durchführung 3, mit Noppen 10 und einem unteren Rand 8 angeordnet, wobei die untere Wandung 2 analog dem ersten Ausführungsbeispiel aufgebaut ist.

Die obere Wandung 31 und die untere Wandung 2 begrenzen einen Hohlraum 34 im Inneren des Implantats. Analog dem ersten Ausführungsbeispiel ist in dem Hohlraum 34 ein Durchstechschutz 6 angeordnet, wie er in Figur 20 gezeigt ist.

Ein oberer Rand 47 der oberen Wandung 31 kann einen Kreis bilden, der die obere Wandung 31 umschließt.

Die obere Wandung 31 und die untere Wandung 2 können über den oberen Rand 47 und den unteren Rand 8 miteinander flüssigkeitsdicht verbunden sein, so dass der Hohlraum 34 bis auf die Durchführung 3 flüssigkeitsdicht verschlossen ist. Hierzu kann an dem oberen Rand 47 ein Rastring 44 ausgebildet sein und an dem unteren Rand 8 ein Rastring 16. Der Rastring 44 des oberen Rands 47 kann in den Rastring 16 des unteren Rands 8 greifen, wenn die obere Wandung 31 vollständig auf die untere Wandung 2 aufgesteckt wird.

Die Verwendung eines solchen Implantats und damit ein erfindungsgemäßes Verfahren erfolgt analog dem ersten Ausführungsbeispiel.

Ein erfindungsgemäßes Verfahren ist im Detail zu den Figuren 24 bis 33 im Zusammenhang mit dem neunten beispielhaften Implantat weiter unten beschrieben. Das Verfahren lässt sich aber ohne weiteres auch auf das eben beschriebene Implantat übertragen.

Das Implantat nach dem fünften Ausführungsbeispiel nach den Figuren 14 und 15 unterscheidet sich von dem zweiten Ausführungsbeispiel durch eine gewölbte obere Wandung 31. Ansonsten gleicht das fünfte Implantat dem zweiten Implantat nach den Figuren 6 bis 8, weist also analog dem zweiten Implantat zwei seitliche Laschen 50 an einem oberen Rand 57 der oberen Wandung 31 auf, mit denen das fünfte Implantat an Weichgewebe angenäht und dort fixiert werden kann. Die obere Wandung 31 ist mit einer üblichen medizinischen Injektionskanüle mit manueller Kraft durchstechbar (so wie das für das neunte Ausführungsbeispiel in den Figuren 27 bis 33 gezeigt ist). Die obere Wandung 31 kann eine Oberseite des Implantats bilden. Das Material für die obere Wandung 31 kann so beschaffen sein, dass die obere Wandung 31 sich nach dem Herausziehen einer durchgestochenen Injektionskanüle wieder selbsttätig verschließt. Hierzu kann die obere Wandung aus einem gummielastischen Kunststoff oder aus Gummi bestehen beziehungsweise zumindest in einem zentralen Bereich einen gummielastischen Kunststoff oder Gummi aufweisen. Derartige selbstdichtende Membranen kommen beispielsweise bei Fläschchen zum Aufziehen von Spritzen zum Einsatz.

Auf der der Oberseite gegenüberliegenden Unterseite des Implantats ist eine untere Wandung 2 mit einer Durchführung 3, mit Noppen 10 und einem unteren Rand 8 angeordnet, wobei die untere Wandung 2 analog dem ersten und zweiten Ausführungsbeispiel aufgebaut ist.

Die obere Wandung 31 und die untere Wandung 2 begrenzen einen Hohlraum 34 im Inneren des Implantats. Analog dem ersten Ausführungsbeispiel ist in dem Hohlraum 34 ein Durchstechschutz 6 angeordnet, wie er in Figur 20 gezeigt ist.

Der obere Rand 57 der oberen Wandung 31 kann einen Kreis bilden, der die obere Wandung 31 umschließt. Die obere Wandung 31 und die untere Wandung 2 können über den oberen Rand 57 und den unteren Rand 8 miteinander flüssigkeitsdicht verbunden sein, so dass der Hohlraum 34 bis auf die Durchführung 3 flüssigkeitsdicht verschlossen ist. Hierzu kann an dem oberen Rand 57 ein Rastring 54 ausgebildet sein und an dem unteren Rand 8 ein Rastring 16. Der Rastring 54 des oberen Rands 57 kann in den Rastring 16 des unteren Rands 8 greifen, wenn die obere Wandung 31 vollständig auf die untere Wandung 2 aufgesteckt wird.

Die Verwendung eines solchen Implantats und damit ein erfindungsgemäßes Verfahren erfolgt analog dem zweiten Ausführungsbeispiel.

Ein erfindungsgemäßes Verfahren ist im Detail zu den Figuren 24 bis 33 im Zusammenhang mit dem neunten beispielhaften Implantat weiter unten beschrieben. Das Verfahren lässt sich aber ohne weiteres auch auf das eben beschriebene Implantat übertragen.

Das Implantat nach dem sechsten Ausführungsbeispiel nach den Figuren 16 und 17 unterscheidet sich von dem fünften Ausführungsbeispiel durch eine untere Wandung 62 mit einem Rohr 69 an der Unterseite der unteren Wandung 62. Ansonsten gleicht das sechste Implantat dem fünften Implantat nach den Figuren 14 bis 15, weist also analog dem fünften Implantat zwei seitliche Laschen 50 an einem oberen Rand 57 der oberen Wandung 31 auf, mit denen das sechste Implantat an Weichgewebe angenäht und dort fixiert werden kann. Die obere Wandung 31 ist mit einer üblichen medizinischen Injektionskanüle mit manueller Kraft durchstechbar (so wie das für das neunte Ausführungsbeispiel in den Figuren 27 bis 33 gezeigt ist). Die obere Wandung 31 kann eine Oberseite des Implantats bilden. Das Material für die obere Wandung 31 kann so beschaffen sein, dass die obere Wandung 31 sich nach dem Herausziehen einer durchgestochenen Injektionskanüle wieder selbsttätig verschließt. Hierzu kann die obere Wandung aus einem gummielastischen Kunststoff oder aus Gummi bestehen beziehungsweise zumindest in einem zentralen Bereich einen gummielastischen Kunststoff oder Gummi aufweisen. Derartige selbstdichtende Membranen kommen beispielsweise bei Fläschchen zum Aufziehen von Spritzen zum Einsatz.

Auf der der Oberseite gegenüberliegenden Unterseite des Implantats ist die untere Wandung 62 mit einer Durchführung 63 angeordnet, wobei auch mehrere Durchführungen (nicht gezeigt) vorgesehen sein können. Die Durchführung 63 ist zylindrisch und reicht durch das Rohr 69. Die Durchführung 63 und das Rohr 69 können zentral in der Mitte der unteren Wandung 62 angeordnet sein. Die Durchführung 63 verbindet die Oberseite der unteren Wandung 62 mit der Unterseite der oberen Wandung 62 und damit mit der Unterseite des Implantats.

Die obere Wandung 31 und die untere Wandung 62 begrenzen einen Hohlraum 34 im Inneren des Implantats. Die Durchführung 63 verbindet den Hohlraum 34 des Implantats mit der Umgebung des Implantats flüssigkeitsdurchlässig. Ein Innenraum 35, der ein Teilstück des Hohlraums 34 ist, kann zwischen einem Durchstechschutz 6 im Hohlraum 34 und der oberen Wandung 31 angeordnet sein. Der Durchstechschutz 6 ist scheibenförmig und ist im Hohlraum 34 zwischen der unteren Wandung 62 und der oberen Wandung 31 angeordnet. Der Durchstechschutz 6 kann scheibenförmig sein. Der Durchstechschutz 6 kann einen kleineren Durchmesser aufweisen als der Hohlraum 34, so dass er den Hohlraum 34 nicht flüssigkeitsdicht in zwei Teile trennt, beziehungsweise in zwei, miteinander flüssigkeitsdurchlässig verbundene Bereiche trennt. Der Durchstechschutz 6 ist im Detail in Figur 20 gezeigt. Der Durchstechschutz 6 ist nicht mit einer üblichen medizinischen Injektionskanüle mit manueller Kraft durchstechbar (so wie das für das neunte Ausführungsbeispiel in den Figuren 27 bis 33 gezeigt ist).

Der obere Rand 57 der oberen Wandung 31 kann einen Kreis bilden, der die obere Wandung 31 umschließt. Die untere Wandung 62 kann einen umlaufenden unteren Rand 8 aufweisen. Der untere Rand 8 der unteren Wandung 62 kann einen Kreis bilden, der die untere Wandung 62 umschließt.

Die untere Wandung 62 kann an einer Unterseite Noppen 60 aufweisen, die von der Unterseite der unteren Wandung 62 abstehen. Auf diese Weise kann sichergestellt werden, dass das Implantat, wenn es mit der Unterseite der unteren Wandung 62 um das Rohr 69 herum auf einer Unterlage aufliegt, dort einen Abstand einhält. Durch den so entstehenden Zwischenraum kann sich dann die Flüssigkeit, die durch die Durchführung 63 und durch das Rohr 69 aus dem Hohlraum 34 austritt, entlang der Oberfläche der unteren Wandung 62 verteilen. Mit dem Rohr 69 kann die Flüssigkeit in einer Vertiefung in der Auflage, wie beispielsweise in einer Kavität an einem Gelenk appliziert werden.

Der Durchstechschutz 6 kann eine wellenförmige Form aufweisen, die an der Oberseite und an seiner Unterseite des Durchstechschutzes 6 abstehende Strukturierungen 12 bildet (siehe Figur 20). Hiermit kann erreicht werden, dass eine Unterseite der oberen Wandung 31 von einer Oberseite des Durchstechschutzes 6 derart beabstandet ist, dass die Spitze einer Injektionskanüle in den Innenraum 35 eindringen kann, um dort die Flüssigkeit zu injizieren. Zudem kann so eine Oberseite der unteren Wandung 62 von der Unterseite des Durchstechschutzes 6 beabstandet sein, so dass eine Flüssigkeit sich problemlos in dem unteren Teil des Innenraums 34 ausbreiten kann und bis zu der Durchführung 63 ausbreiten kann.

Die obere Wandung 31 und die untere Wandung 62 können über den oberen Rand 57 und den unteren Rand 8 miteinander flüssigkeitsdicht verbunden sein, so dass der Hohlraum 34 bis auf die Durchführung 63 flüssigkeitsdicht verschlossen ist. Hierzu kann an dem oberen Rand 57 ein Rastring 54 ausgebildet sein und an dem unteren Rand 8 ein Rastring 16. Der Rastring 54 des oberen Rands 57 kann in den Rastring 16 des unteren Rands 8 greifen, wenn die obere Wandung 31 vollständig auf die untere Wandung 62 aufgesteckt wird.

Die Verwendung eines solchen Implantats und damit ein erfindungsgemäßes Verfahren können dadurch erfolgen, dass eine Injektionskanüle einer Spritze in einem mittleren Bereich durch die obere Wandung 31 gestochen wird. Anschließend kann eine Flüssigkeit aus der Spritze in den Innenraum 35 injiziert werden. Der Durchstechschutz 6 kann mit Hilfe von in den Hohlraum 34 ragenden Nasen (nicht zu sehen, aber analog den Nasen 18 nach dem ersten Ausführungsbeispiel) zentral gehalten werden, so dass der Durchstechschutz 6 seitlich von dem unteren Rand 8 beabstandet ist (analog den Figuren 2 und 4 und Figur 5 unten). Die Nasen können allseitig an einem inneren Umfang des unteren Rands 8 angeordnet sein. Die Flüssigkeit kann so von dem Innenraum 35 in den unteren Teil des Hohlraums 34 zwischen dem Durchstechschutz 6 und der unteren Wandung 62 fließen.

Die untere Wandung 62 und die obere Wandung 31 können elastisch expandierbar sein. Durch das Einspritzen der Flüssigkeit in den Hohlraum 34 kann der Hohlraum 34 dann elastisch verformt, beziehungsweise elastisch expandiert werden. Die Flüssigkeit in dem Hohlraum 34 steht dann unter einem elastischen Druck. Dadurch kann die Flüssigkeit durch die Durchführung 63 aus dem Hohlraum 34 ausgepresst werden. Das Implantat kann zuvor oder auch danach subkutan im Bereich eines Gelenks oder an einer anderen zu behandelnden Stelle Implantiert werden und mit Hilfe der Laschen 50 an Weichgewebe angenäht und fixiert werden. Die Flüssigkeit kann sich nach dem Austreten aus dem Hohlraum 34 durch die Durchführung 63, das Rohr 69 entlang und entlang des Zwischenraums zwischen der Unterseite der unteren Wandung 62 um das Rohr 69 herum und der Auflage ausbreiten und verteilen. Wenn keine Flüssigkeit mehr aus dem Hohlraum 34 herausfließt, kann entweder ein Druck auf das Implantat ausgeübt werden, um einen Gegendruck zu überwinden und weitere Flüssigkeit aus dem Implantat freizusetzen oder es kann neue Flüssigkeit mit einer Spritze in den Innenraum 35 injiziert werden.

Ein erfindungsgemäßes Verfahren ist im Detail zu den Figuren 24 bis 33 im Zusammenhang mit dem neunten beispielhaften Implantat weiter unten beschrieben. Das Verfahren lässt sich aber ohne weiteres auch auf das eben beschriebene Implantat übertragen.

Das Implantat nach dem siebten Ausführungsbeispiel nach den Figuren 18 und 19 unterscheidet sich von dem sechsten Ausführungsbeispiel nach den Figuren 16 und 17 durch radiale Stege 70 anstelle von Noppen 60. Ansonsten gleicht das siebte Implantat dem sechsten Implantat nach den Figuren 16 und 17, weist also analog dem sechsten Implantat ein Rohr 79 zum Formen einer Durchführung 73 durch eine untere Wandung 72 auf und weist zwei seitliche Laschen 50 an einem oberen Rand 57 der oberen Wandung 31 auf, mit denen das fünfte Implantat an Weichgewebe angenäht und dort fixiert werden kann. Die obere Wandung 31 ist mit einer üblichen medizinischen Injektionskanüle mit manueller Kraft durchstechbar (so wie das für das neunte Ausführungsbeispiel in den Figuren 27 bis 33 gezeigt ist). Die obere Wandung 31 kann eine Oberseite des Implantats bilden. Das Material für die obere Wandung 31 kann so beschaffen sein, dass die obere Wandung 31 sich nach dem Herausziehen einer durchgestochenen Injektionskanüle wieder selbsttätig verschließt. Hierzu kann die obere Wandung aus einem gummielastischen Kunststoff oder aus Gummi bestehen beziehungsweise zumindest in einem zentralen Bereich einen gummielastischen Kunststoff oder Gummi aufweisen. Derartige selbstdichtende Membranen kommen beispielsweise bei Fläschchen zum Aufziehen von Spritzen zum Einsatz.

Auf der der Oberseite gegenüberliegenden Unterseite des Implantats ist die untere Wandung 72 mit dem Rohr 79 und der Durchführung 73, mit Stegen 70 und einem unteren Rand 8 angeordnet. Die Flüssigkeit aus dem Implantat kann entlang der Stegen 70 radial nach außen geleitet werden. Die Stegen 70 sichern dabei einen freien Zwischenraum zwischen einer Auflage des Implantats und der Unterseite des Implantats.

Die obere Wandung 31 und die untere Wandung 72 begrenzen einen Hohlraum 34 im Inneren des Implantats. Analog dem ersten Ausführungsbeispiel ist in dem Hohlraum 34 ein Durchstechschutz 6 angeordnet, der in Figur 20 gezeigt ist.

Die Verwendung eines solchen Implantats und damit ein erfindungsgemäßes Verfahren erfolgt analog dem sechsten Ausführungsbeispiel.

Ein erfindungsgemäßes Verfahren ist im Detail zu den Figuren 24 bis 33 im Zusammenhang mit dem neunten beispielhaften Implantat weiter unten beschrieben. Das Verfahren lässt sich aber ohne weiteres auch auf das eben beschriebene Implantat übertragen.

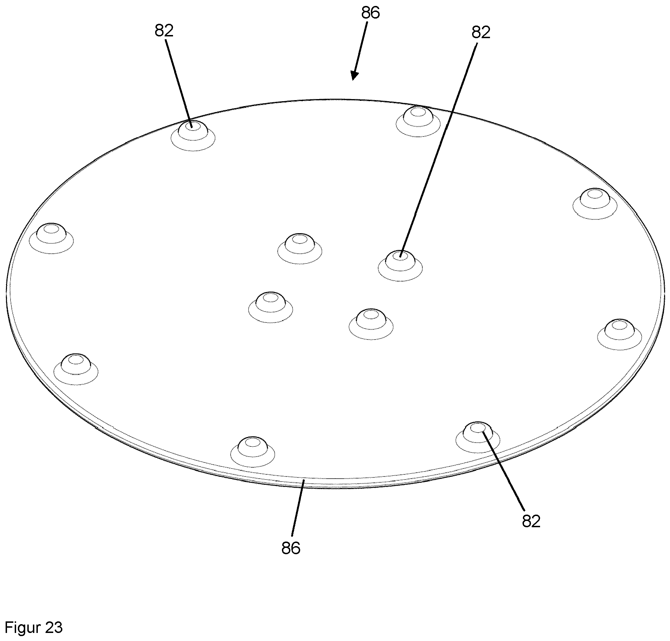

Das Implantat nach dem achten Ausführungsbeispiel nach den Figuren 21 bis 23 unterscheidet sich von dem fünften Ausführungsbeispiel durch einen abgewandelten Durchstechschutz 86, der in Figur 23 gezeigt ist. Ansonsten gleicht das achte Implantat dem fünften Implantat nach den Figuren 14 bis 15, weist also analog dem fünften Implantat zwei seitliche Laschen 50 an einem oberen Rand 57 der oberen Wandung 31 auf, mit denen das achte Implantat an Weichgewebe angenäht und dort fixiert werden kann. Die obere gewölbte Wandung 31 ist mit einer üblichen medizinischen Injektionskanüle mit manueller Kraft durchstechbar (so wie das für das neunte Ausführungsbeispiel in den Figuren 27 bis 33 gezeigt ist). Die obere Wandung 31 kann eine Oberseite des Implantats bilden. Das Material für die obere Wandung 31 kann so beschaffen sein, dass die obere Wandung 31 sich nach dem Herausziehen einer durchgestochenen Injektionskanüle wieder selbsttätig verschließt. Hierzu kann die obere Wandung aus einem gummielastischen Kunststoff oder aus Gummi bestehen beziehungsweise zumindest in einem zentralen Bereich einen gummielastischen Kunststoff oder Gummi aufweisen. Derartige selbstdichtende Membranen kommen beispielsweise bei Fläschchen zum Aufziehen von Spritzen zum Einsatz.

Auf der der Oberseite gegenüberliegenden Unterseite des Implantats ist die untere Wandung 2 mit einer Durchführung 3 angeordnet, wobei auch mehrere Durchführungen (nicht gezeigt) vorgesehen sein können. Die Durchführung 3 kann zentral in der Mitte der unteren Wandung 2 angeordnet sein. Die Durchführung 3 verbindet die Oberseite der unteren Wandung 2 mit der Unterseite der oberen Wandung 2 und damit mit der Unterseite des Implantats.

Die obere Wandung 31 und die untere Wandung 2 begrenzen einen Hohlraum 84 im Inneren des Implantats. Die Durchführung 3 verbindet den Hohlraum 84 des Implantats mit der Umgebung des Implantats flüssigkeitsdurchlässig. Ein Innenraum 85, der ein Teilstück des Hohlraums 84 ist, kann zwischen dem Durchstechschutz 86 im Hohlraum 84 und der oberen Wandung 31 angeordnet sein. Der Durchstechschutz 86 ist scheibenförmig und ist im Hohlraum 84 zwischen der unteren Wandung 2 und der oberen Wandung 31 angeordnet. Der Durchstechschutz 86 kann scheibenförmig sein und an seiner Unterseite eine vorspringende Oberflächenstrukturierung 82 aufweisen. Der Durchstechschutz 86 kann einen kleineren Durchmesser aufweisen als der Hohlraum 84, so dass er den Hohlraum 84 nicht flüssigkeitsdicht in zwei Teile trennt, beziehungsweise in zwei, miteinander flüssigkeitsdurchlässig verbundene Bereiche trennt. Der Durchstechschutz 86 ist im Detail in Figur 23 gezeigt. Der Durchstechschutz 86 ist nicht mit einer üblichen medizinischen Injektionskanüle mit manueller Kraft durchstechbar (so wie das für das neunte Ausführungsbeispiel in den Figuren 27 bis 33 gezeigt ist).

Der obere Rand 57 der oberen Wandung 31 kann einen Kreis bilden, der die obere Wandung 31 umschließt. Die untere Wandung 2 kann einen umlaufenden unteren Rand 8 aufweisen. Der untere Rand 8 der unteren Wandung 2 kann einen Kreis bilden, der die untere Wandung 2 umschließt.

Die untere Wandung 2 kann an einer Unterseite Noppen 10 aufweisen, die von der Unterseite der unteren Wandung 2 abstehen. Auf diese Weise kann sichergestellt werden, dass das Implantat, wenn es mit der Unterseite der unteren Wandung 2 auf einer Unterlage aufliegt, dort einen Abstand einhält. Durch den so entstehenden Zwischenraum kann sich dann die Flüssigkeit, die durch die Durchführung 3 aus dem Hohlraum 84 austritt, entlang der Oberfläche der unteren Wandung 2 verteilen.

Der Durchstechschutz 86 kann eine ebene Form aufweisen mit von der Unterseite des Durchstechschutzes 86 abstehenden Oberflächenstrukturierungen 82 (siehe Figur 23). Hiermit kann erreicht werden, dass eine Oberseite der unteren Wandung 2 von der Unterseite des Durchstechschutzes 86 beabstandet ist, so dass eine Flüssigkeit sich problemlos in dem unteren Teil des Innenraums 84 ausbreiten kann und bis zu der Durchführung 3 ausbreiten kann.

Die obere Wandung 31 und die untere Wandung 2 können über den oberen Rand 57 und den unteren Rand 8 miteinander flüssigkeitsdicht verbunden sein, so dass der Hohlraum 84 bis auf die Durchführung 3 flüssigkeitsdicht verschlossen ist. Hierzu kann an dem oberen Rand 57 ein Rastring 54 ausgebildet sein und an dem unteren Rand 8 ein Rastring 16. Der Rastring 54 des oberen Rands 57 kann in den Rastring 16 des unteren Rands 8 greifen, wenn die obere Wandung 31 vollständig auf die untere Wandung 2 aufgesteckt wird.

Die Verwendung eines solchen Implantats und damit ein erfindungsgemäßes Verfahren können dadurch erfolgen, dass eine Injektionskanüle einer Spritze in einem mittleren Bereich durch die obere Wandung 31 gestochen wird. Anschließend kann eine Flüssigkeit aus der Spritze in den Innenraum 85 injiziert werden. Der Durchstechschutz 86 kann mit Hilfe von in den Hohlraum 84 ragenden Nasen (nicht zu sehen, aber analog den Nasen 18 nach dem ersten Ausführungsbeispiel) zentral gehalten werden, so dass der Durchstechschutz 86 seitlich von dem unteren Rand 8 beabstandet ist (analog den Figuren 2 und 4 und Figur 5 unten). Die Nasen können allseitig an einem inneren Umfang des unteren Rands 8 angeordnet sein. Die Flüssigkeit kann so von dem Innenraum 85 in den unteren Teil des Hohlraums 84 zwischen dem Durchstechschutz 86 und der unteren Wandung 2 fließen.

Die untere Wandung 2 und die obere Wandung 31 können elastisch expandierbar sein. Durch das Einspritzen der Flüssigkeit in den Hohlraum 84 kann der Hohlraum 84 dann elastisch verformt, beziehungsweise elastisch expandiert werden. Die Flüssigkeit in dem Hohlraum 84 steht dann unter einem elastischen Druck. Dadurch kann die Flüssigkeit durch die Durchführung 3 aus dem Hohlraum 84 ausgepresst werden. Das Implantat kann zuvor oder auch danach subkutan im Bereich eines Gelenks oder an einer anderen zu behandelnden Stelle Implantiert werden und mit Hilfe der Laschen 50 an Weichgewebe angenäht und fixiert werden. Die Flüssigkeit kann sich nach dem Austreten aus dem Hohlraum 84 durch die Durchführung 3 und entlang des Zwischenraums zwischen der Unterseite der unteren Wandung 2 und der Auflage ausbreiten und verteilen. Wenn keine Flüssigkeit mehr aus dem Hohlraum 84 herausfließt, kann entweder ein Druck auf das Implantat ausgeübt werden, um einen Gegendruck zu überwinden und weitere Flüssigkeit aus dem Implantat freizusetzen oder es kann neue Flüssigkeit mit einer Spritze in den Innenraum 85 injiziert werden.

Ein erfindungsgemäßes Verfahren ist im Detail zu den Figuren 24 bis 33 im Zusammenhang mit dem neunten beispielhaften Implantat weiter unten beschrieben. Das Verfahren lässt sich aber ohne weiteres auch auf das eben beschriebene Implantat übertragen.

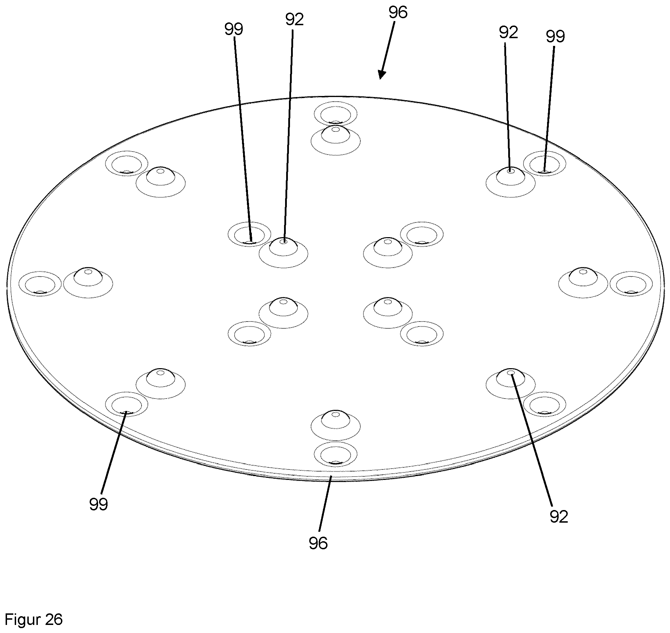

Das Implantat nach dem neunten Ausführungsbeispiel nach den Figuren 24 bis 26 unterscheidet sich von dem achten Ausführungsbeispiel durch einen abgewandelten Durchstechschutz 96, der in Figur 26 gezeigt ist. Ansonsten gleicht das neunte Implantat dem achten Implantat nach den Figuren 21 bis 23, weist also analog dem achten Implantat zwei seitliche Laschen 50 an einem oberen Rand 57 der oberen Wandung 31 auf, mit denen das neunte Implantat an Weichgewebe angenäht und dort fixiert werden kann. Der Ablauf eines erfindungsgemäßen Verfahrens ist in den Figuren 27 bis 33 anhand des neunten Ausführungsbeispiels erläutert.

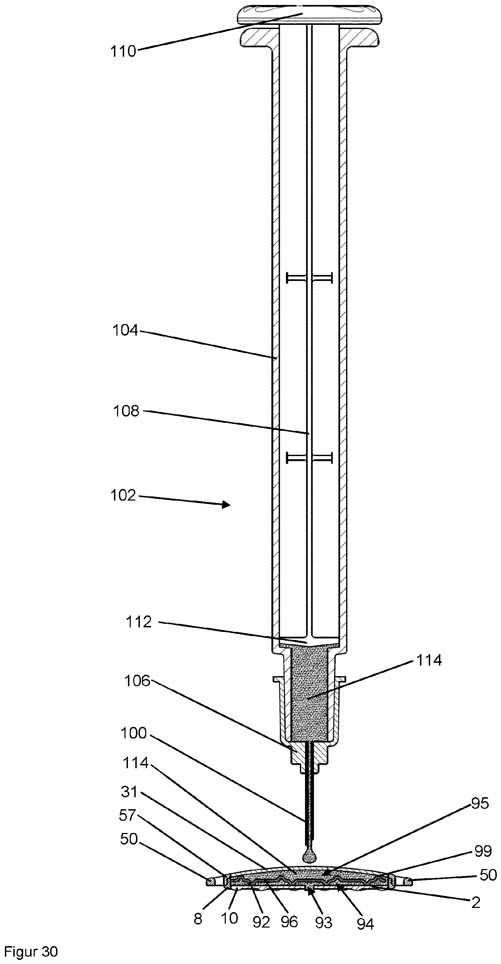

Die obere gewölbte Wandung 31 ist mit einer üblichen medizinischen Injektionskanüle 100 mit manueller Kraft durchstechbar, so wie das in den Figuren 27 bis 33 gezeigt ist. Die obere Wandung 31 kann eine Oberseite des Implantats bilden. Das Material für die obere Wandung 31 kann so beschaffen sein, dass die obere Wandung 31 sich nach dem Herausziehen der durchgestochenen medizinischen Injektionskanüle 100 wieder selbsttätig verschließt (siehe Figur 30). Hierzu kann die obere Wandung aus einem gummielastischen Kunststoff oder aus Gummi bestehen beziehungsweise zumindest in einem zentralen Bereich einen gummielastischen Kunststoff oder Gummi aufweisen. Derartige selbstdichtende Membranen kommen beispielsweise bei Fläschchen zum Aufziehen von Spritzen zum Einsatz.

Auf der der Oberseite gegenüberliegenden Unterseite des Implantats ist die untere Wandung 2 mit einer Durchführung 3 angeordnet, wobei auch mehrere Durchführungen (nicht gezeigt) vorgesehen sein können. Die Durchführung 3 kann zentral in der Mitte der unteren Wandung 2 angeordnet sein. Die Durchführung 3 verbindet die Oberseite der unteren Wandung 2 mit der Unterseite der oberen Wandung 2 und damit mit der Unterseite des Implantats.

Die obere Wandung 31 und die untere Wandung 2 begrenzen einen Hohlraum 94 im Inneren des Implantats. Die Durchführung 3 verbindet den Hohlraum 94 des Implantats mit der Umgebung des Implantats flüssigkeitsdurchlässig. Ein Innenraum 95, der ein Teilstück des Hohlraums 94 ist, kann zwischen dem Durchstechschutz 96 im Hohlraum 94 und der oberen Wandung 31 angeordnet sein. Der Durchstechschutz 96 ist scheibenförmig und ist im Hohlraum 94 zwischen der unteren Wandung 2 und der oberen Wandung 31 angeordnet. Der Durchstechschutz 96 kann scheibenförmig sein und an seiner Unterseite eine vorspringende Oberflächenstrukturierung 92 aufweisen sowie an seiner Oberseite eine vorspringende Oberflächenstrukturierung 99 aufweisen. Der Durchstechschutz 96 kann einen kleineren Durchmesser aufweisen als der Hohlraum 94, so dass er den Hohlraum 94 nicht flüssigkeitsdicht in zwei Teile trennt, beziehungsweise in zwei, miteinander flüssigkeitsdurchlässig verbundene Bereiche trennt. Der Durchstechschutz 96 ist im Detail in Figur 26 gezeigt. Der Durchstechschutz 96 ist nicht mit der medizinischen Injektionskanüle 100 einer Spritze 102 mit manueller Kraft durchstechbar, so wie das in den Figuren 27 bis 33 gezeigt ist.

Der obere Rand 57 der oberen Wandung 31 kann einen Kreis bilden, der die obere Wandung 31 umschließt. Die untere Wandung 2 kann einen umlaufenden unteren Rand 8 aufweisen. Der untere Rand 8 der unteren Wandung 2 kann einen Kreis bilden, der die untere Wandung 2 umschließt.

Die untere Wandung 2 kann an einer Unterseite Noppen 10 aufweisen, die von der Unterseite der unteren Wandung 2 abstehen. Auf diese Weise kann sichergestellt werden, dass das Implantat, wenn es mit der Unterseite der unteren Wandung 2 auf einer Unterlage aufliegt, dort einen Abstand einhält. Durch den so entstehenden Zwischenraum kann sich dann die Flüssigkeit, die durch die Durchführung 3 aus dem Hohlraum 94 austritt, entlang der Oberfläche der unteren Wandung 2 verteilen.

Der Durchstechschutz 96 kann eine ebene Form aufweisen mit von der Unterseite des Durchstechschutzes 96 abstehenden Oberflächenstrukturierungen 92 mit von der Oberseite des Durchstechschutzes 96 abstehenden Oberflächenstrukturierungen 99 (siehe Figur 26). Mit der Oberflächenstrukturierung 99 an der Oberseite des Durchstechschutzes 96 kann erreicht werden, dass eine Unterseite der oberen Wandung 31 von einer Oberseite des Durchstechschutzes 96 derart beabstandet ist, dass die Spitze der Injektionskanüle 100 in den Innenraum 95 eindringen kann, um dort die Flüssigkeit zu injizieren. Zudem kann mit den Oberflächenstrukturierungen 92 der Unterseite des Durchstechschutzes 96 eine Oberseite der unteren Wandung 2 von der Unterseite des Durchstechschutzes 96 beabstandet werden, so dass eine Flüssigkeit sich problemlos in dem unteren Teil des Innenraums 94 ausbreiten kann und bis zu der Durchführung 3 ausbreiten kann.

Die obere Wandung 31 und die untere Wandung 2 können über den oberen Rand 57 und den unteren Rand 8 miteinander flüssigkeitsdicht verbunden sein, so dass der Hohlraum 94 bis auf die Durchführung 3 flüssigkeitsdicht verschlossen ist. Hierzu kann an dem oberen Rand 57 ein Rastring 54 ausgebildet sein und an dem unteren Rand 8 ein Rastring 16. Der Rastring 54 des oberen Rands 57 kann in den Rastring 16 des unteren Rands 8 greifen, wenn die obere Wandung 31 vollständig auf die untere Wandung 2 aufgesteckt wird.

Im Folgenden wird der Ablauf eines erfindungsgemäßen Verfahrens anhand des neunten Implantats und anhand der Figuren 24 bis 33 erläutert. Die Spritze 102 und das nicht mit Flüssigkeit gefüllte Implantat werden bereitgestellt (siehe Figur 27). Die Spritze 102 weist an ihrer Vorderseite die medizinische Injektionskanüle 100 auf. Ein durch eine Kartusche 104 der Spritze 102 begrenzter Innenraum kann durch die Injektionskanüle 100 mit einer Flüssigkeit 114 gefüllt werden. Die Injektionskanüle 100 ist über einen Kartuschenkopf 106 mit der Kartusche 104 verbunden. Der Inhalt der Spritze 102 kann mit Hilfe eines Kolbens 112, der über einen Stößel 108 mit einem Griff 110 in der Kartusche 104 in Richtung der Injektionskanüle 100 vorgetrieben werden kann, aus der Kartusche 104 durch die Injektionskanüle 100 appliziert werden.

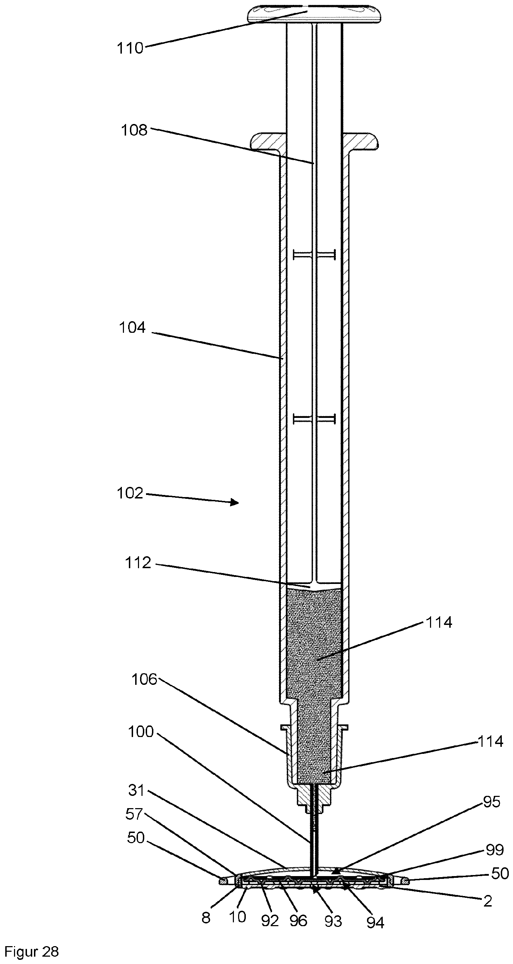

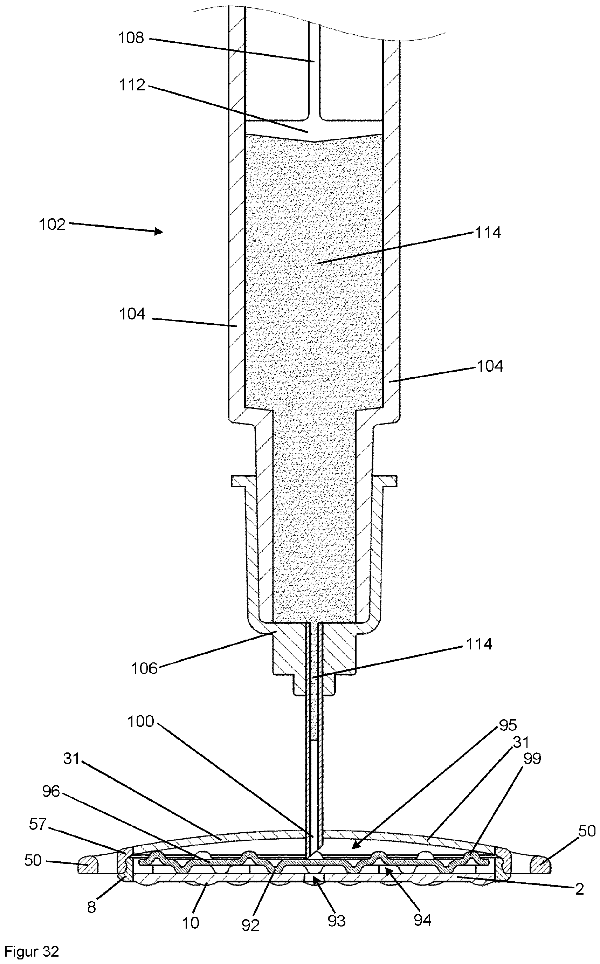

Die Injektionskanüle 100 der mit der Flüssigkeit 114 gefüllten Spritze 102 wird in einem mittleren Bereich durch die obere Wandung 31 gestochen (Figuren 28 und 32). Die Flüssigkeit 114 enthält wenigstens einen pharmazeutischen Wirkstoff, insbesondere Cyclosporin A.

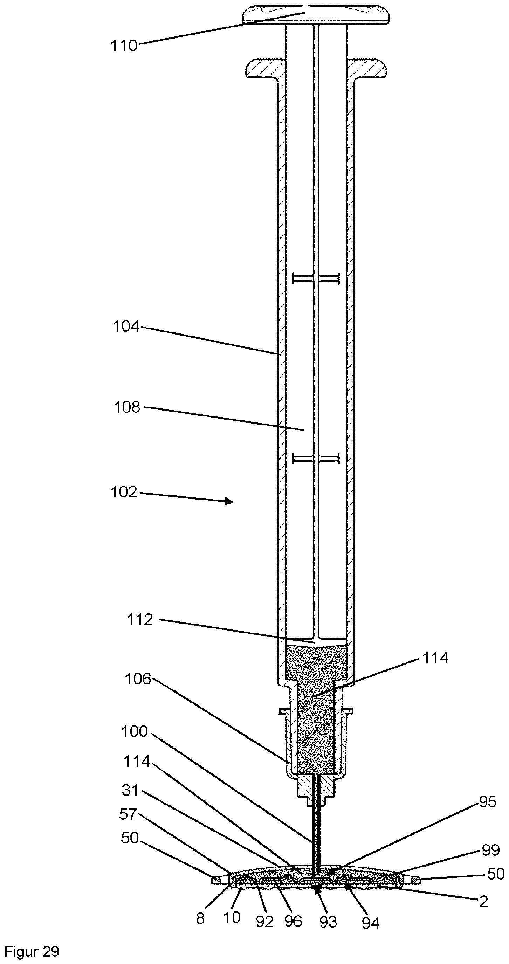

Anschließend wird die Flüssigkeit 114 aus der Spritze 102 in den Innenraum 95 injiziert (siehe Figur 29), indem der Kolben 112 mit Hilfe des Griffs 110 und des Stößels 108 in Richtung der Injektionskanüle 100 gedrückt wird. Der Durchstechschutz 96 kann mit Hilfe von in den Hohlraum 94 ragenden Nasen (nicht zu sehen, aber analog den Nasen 18 nach dem ersten Ausführungsbeispiel) zentral gehalten werden, so dass der Durchstechschutz 96 seitlich von dem unteren Rand 8 beabstandet ist (analog den Figuren 2 und 4 und Figur 5 unten). Die Nasen können allseitig an einem inneren Umfang des unteren Rands 8 angeordnet sein. Die Flüssigkeit kann so von dem Innenraum 95 in den unteren Teil des Hohlraums 94 zwischen dem Durchstechschutz 96 und der unteren Wandung 2 fließen. Alternativ oder zusätzlich können auch Durchbrechungen (nicht gezeigt) in dem Durchstechschutz 96 vorgesehen sein, durch die die Flüssigkeit von dem Innenraum 95 in den unteren Teil des Hohlraum 94 fließen kann. Diese Modifikation kann auch bei allen anderen Ausführungsbeispielen angewendet werden.

Die untere Wandung 2 und die obere Wandung 31 können elastisch expandierbar sein. Durch das Einspritzen der Flüssigkeit 114 in den Hohlraum 94 kann der Hohlraum 94 dann elastisch verformt, beziehungsweise elastisch expandiert werden. Die Flüssigkeit 114 in dem Hohlraum 84 steht dann unter einem elastischen Druck. Wenn der Hohlraum 94 mit der Flüssigkeit gefüllt ist, kann die Injektionskanüle 100 aus der oberen Wandung 31 herausgezogen werden. Die obere Wandung 31 verschließt sich dabei selbstständig. Dies ist in Figur 30 gezeigt.

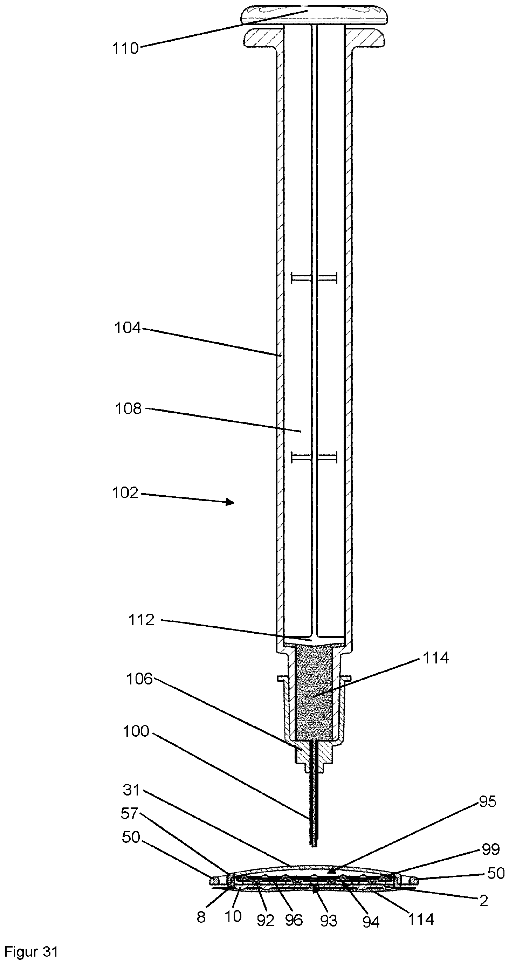

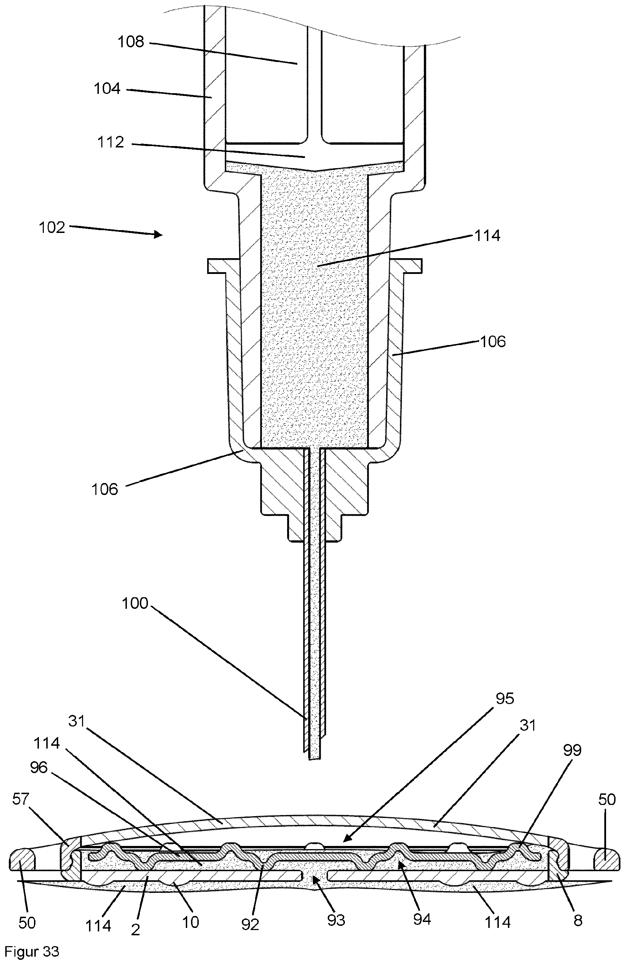

Durch den elastischen Druck der oberen Wandung 31 und der unteren Wandung kann die Flüssigkeit 114 durch die Durchführung 3 aus dem Hohlraum 84 ausgepresst werden und nach unten durch die Durchführung 3 ausfließen (siehe Figuren 31 und 33). Das Implantat kann zuvor oder auch danach subkutan im Bereich eines Gelenks oder an einer anderen zu behandelnden Stelle Implantiert werden und mit Hilfe der Laschen 50 an Weichgewebe angenäht und fixiert werden. Die Flüssigkeit 114 kann sich nach dem Austreten aus dem Hohlraum 94 durch die Durchführung 3 und entlang des Zwischenraums zwischen der Unterseite der unteren Wandung 2 und der Auflage ausbreiten und verteilen. Wenn keine Flüssigkeit 114 mehr aus dem Hohlraum 94 herausfließt, kann entweder ein Druck auf das Implantat ausgeübt werden, um einen Gegendruck zu überwinden und weiter Flüssigkeit 114 aus dem Implantat freizusetzen oder es kann neue Flüssigkeit 114 mit der Spritze 102 in den Innenraum 95 injiziert werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 31: Obere Wandung
- 2, 62, 72: Untere Wandung
- 3, 63, 73: Durchführung
- 4, 34, 84, 94: Hohlraum
- 5, 35, 85, 95: Innenraum
- 6, 86, 96: Durchstechschutz
- 7, 27, 37, 47, 57: Oberer Rand
- 8: Unterer Rand
- 10,60: Noppe
- 12: Abstehende Strukturierung
- 14, 24, 34, 44, 54: Rastring
- 16: Rastring
- 18: Nase
- 20, 50: Lasche
- 30: Lochring
- 39: Lasche / Loch
- 69, 79: Rohr
- 70: Steg
- 82, 92: Oberflächenstrukturierung
- 99: Oberflächenstrukturierung
- 100: Injektionskanüle
- 102: Spritze
- 104: Kartusche
- 106: Kartuschenkopf
- 108: Stößel
- 110: Griff
- 112: Kolben
- 114: Flüssigkeit

## Patentansprüche

1. Implantat zur lokalen Wirkstofffreisetzung, das Implantat aufweisend
eine obere Wandung (1, 31), wobei die obere Wandung (1, 31) geschlossen und scheibenförmig ist und aus einem Material besteht, das mit einer medizinischen Injektionskanüle (100) mit einer Kraft von weniger als 100 N durchstechbar ist, das selbstdichtend ist und das elastisch verformbar ist,
eine untere Wandung (2, 62, 72), wobei die untere Wandung (2, 62, 72) der oberen Wandung (1, 31) gegenüberliegend angeordnet ist, scheibenförmig ist, elastisch verformbar ist und die untere Wandung (2, 62, 72) zumindest eine Durchführung (3, 63, 73) durch die untere Wandung (2, 62, 72) aufweist, wobei die zumindest eine Durchführung (3, 63, 73) flüssigkeitsdurchlässig ist,
einen Hohlraum (4, 34, 84, 94), der zwischen der oberen Wandung (1, 31) und der unteren Wandung (2, 62, 72) angeordnet ist,
einen Durchstechschutz (6, 86, 96), wobei der Durchstechschutz (6, 86, 96) zwischen der oberen Wandung (1, 31) und der unteren Wandung (2, 62, 72) angeordnet ist und scheibenförmig ist, wobei der Durchstechschutz (6, 86, 96) aus einem Material besteht, das mit einer Kraft von weniger als 100 N nicht mit einer medizinischen Injektionskanüle (100) durchstechbar ist.

2. Implantat nach Anspruch 1, **dadurch gekennzeichnet, dass**
die obere Wandung (1, 31) und die untere Wandung (2, 62, 72) über einen umlaufenden Rand (7, 8, 27, 37, 47, 57) miteinander verbunden sind oder über eine umlaufende Begrenzung des Durchstechschutzes (6, 86, 96) miteinander verbunden sind, wobei bevorzugt die obere Wandung (1, 31) und die untere Wandung (2, 62, 72) flüssigkeitsdicht miteinander verbunden sind.

3. Implantat nach Anspruch 1 oder 2, **dadurch gekennzeichnet, dass**
der Durchstechschutz (6, 86, 96) aus Metall besteht oder zu mindestens 50% aus Metall besteht und/oder
der Durchstechschutz (6, 86, 96) in dem Hohlraum (4, 34, 84, 94) angeordnet ist, wobei bevorzugt der Durchstechschutz (6, 86, 96) nicht fest mit der oberen Wandung (1, 31) verbunden ist und besonders bevorzugt der Durchstechschutz (6, 86, 96) auf einer der oberen Wandung (1, 31) zugewandten Seite der unteren Wandung (2, 62, 72) angeordnet ist.

4. Implantat nach einem der vorangehenden Ansprüche, **dadurch gekennzeichnet, dass** die untere Wandung (2, 62, 72) mit einer medizinischen Injektionskanüle (100) durchstechbar ist, wobei bevorzugt die untere Wandung (2, 62, 72) aus einem gummielastischen Kunststoff besteht, und/oder
das Verhältnis der Breite zur Höhe und der Tiefe zur Höhe des Implantats mindestens 2:1 ist, bevorzugt zumindest 3:1 ist.

5. Implantat nach einem der vorangehenden Ansprüche, **dadurch gekennzeichnet, dass** der Hohlraum (4, 34, 84, 94) durch Injektion einer Flüssigkeit (114) elastisch expandierbar ist, wobei bevorzugt die Flüssigkeit (114) in dem expandierten Hohlraum (4, 34, 84, 94) mit einer elastischen Kraft durch die zumindest eine Durchführung (3, 63, 73) aus dem Hohlraum (4, 34, 84, 94) auspressbar ist.

6. Implantat nach einem der vorangehenden Ansprüche, **dadurch gekennzeichnet, dass** die untere Wandung (2, 62, 72) auf einer der oberen Wandung (1, 31) gegenüberliegenden Seite eine abstehende Strukturierung aufweist, die zur Verteilung einer Flüssigkeit (114) auf dieser äußeren Oberfläche geeignet ist, wobei bevorzugt die abstehende Strukturierung Stege (70) und/oder Noppen (10, 60) sind und/oder die zumindest eine Durchführung (3, 63, 73) in zumindest einen Kanal auf dieser äußeren Oberfläche der unteren Wandung (2, 62, 72) mündet und der zumindest eine Kanal durch die abstehende Strukturierung geformt ist, und/oder
im Hohlraum (4, 34, 84, 94) eine Flüssigkeit (114) enthalten ist, wobei die Flüssigkeit (114) bevorzugt zumindest einen pharmazeutischen Wirkstoff enthält, wobei besonders bevorzugt einer des zumindest einen Wirkstoffs Cyclosporin A ist, oder im Hohlraum (4, 34, 84, 94) eine Wirkstofflösung oder ein im festen oder halbfesten Zustand befindlicher Wirkstoff enthalten ist.

7. Implantat nach einem der vorangehenden Ansprüche, **dadurch gekennzeichnet, dass** der Durchstechschutz (6, 86, 96) mindestens so groß ist wie 50% einer inneren Oberfläche der unteren Wandung (2, 62, 72), die den Hohlraum (4, 34, 84, 94) begrenzt, bevorzugt mindestens so groß ist wie 75% der inneren Oberfläche der unteren Wandung (2, 62, 72), und/oder
der Durchstechschutz (6, 86, 96) an einer umlaufenden Begrenzung des Durchstechschutzes (6, 86, 96) nicht bündig an der Innenseite des Hohlraums (4, 34, 84, 94) anliegt.

8. Implantat nach einem der vorangehenden Ansprüche, **dadurch gekennzeichnet, dass** das Implantat scheibenförmig ist und die obere Wandung (1, 31) eine Oberseite des Implantats bildet und die untere Wandung (2, 62, 72) eine Unterseite des Implantats bildet, wobei bevorzugt die gesamte Oberfläche des Implantats oder die gesamte Oberfläche des Implantats bis auf einen umlaufenden Rand (7, 8, 27, 37, 47, 57) durch die obere Wandung (1, 31) und die untere Wandung (2, 62, 72) gebildet ist, und/oder der Hohlraum (4, 34, 84, 94) einen Innenraum (5, 35, 85, 95) aufweist, der von der oberen Wandung (1, 31) und von dem Durchstechschutz (6, 86, 96) begrenzt ist, wobei bevorzugt durch den Innenraum (5, 35, 85, 95) die obere Wandung (1, 31) von dem Durchstechschutz (6, 86, 96) zumindest 0,5 mm beabstandet ist, besonders bevorzugt zumindest 1 mm beabstandet ist.

9. Implantat nach einem der vorangehenden Ansprüche, **dadurch gekennzeichnet, dass** in jeder der zumindest einen Durchführung (3, 63, 73) jeweils ein Überdruckventil angeordnet ist, das ab einem Mindestdruck nach außen öffnet, bevorzugt ab einem Mindestdruck von mindestens 20 kPa nach außen öffnet, und/oder die obere Wandung (1, 31) und/oder die untere Wandung (2, 62, 72) aus einem gummielastischen Kunststoff besteht oder bestehen oder bis auf einen Rand (7, 8, 27, 37, 47, 57) aus einem gummielastischen Kunststoff besteht oder bestehen, wobei bevorzugt der gummielastische Kunststoff der oberen Wandung (1, 31) sich nach einem Durchstechen und wieder Herausziehen eine medizinischen Injektionskanüle (100) wieder zusammenzieht und flüssigkeitsundurchlässig verschließt.

10. Implantat nach einem der vorangehenden Ansprüche, **dadurch gekennzeichnet, dass** der Durchstechschutz (6, 86, 96) zumindest eine flüssigkeitsdurchlässige Durchbrechung aufweist, wobei bevorzugt die zumindest eine Durchbrechung einen freien Querschnitt von maximal 0,5 mm hat, besonders bevorzugt von maximal 0,25 mm hat, und/oder
der Durchstechschutz (6, 86, 96) auf der der unteren Wandung (2, 62, 72) zugewandten Seite eine Oberflächenstrukturierung (82, 92) aufweist, bevorzugt auf der der oberen Wandung (1, 31) zugewandten Seite und auf der der unteren Wandung (2, 62, 72) zugewandten Seite eine Oberflächenstrukturierung (82, 92, 99) aufweist.

11. Implantat nach einem der vorangehenden Ansprüche, **dadurch gekennzeichnet, dass** der Durchstechschutz (6, 86, 96) mit der unteren Wandung (2, 62, 72) fest verbunden ist und/oder
der Durchstechschutz (6, 86, 96) auf einer Oberseite, die der oberen Wandung (1, 31) zugewandt ist, eine konkave Form hat und/oder eine vorstehende Begrenzung aufweist, und/oder
das Implantat eine oder mehrere Laschen (20, 39, 50) aufweist, an denen das Implantat an Weichgewebe angenäht werden kann.

12. Verfahren zum Befüllen eines Implantats nach einem der vorangehenden Ansprüche, **gekennzeichnet durch** die Schritte
A) Bereitstellen des Implantats und einer mit einer Flüssigkeit (114) gefüllten Spritze (102), wobei die Spritze (102) eine medizinische Injektionskanüle (100) aufweist,
B) Durchstechen der oberen Wandung (1, 31) mit der medizinischen Injektionskanüle (100) der Spritze (102),
C) Einspritzen von Flüssigkeit (114) aus der Spritze (102) durch die medizinische Injektionskanüle (100) in den Hohlraum (4, 34, 84, 94) des Implantats,
D) Ausbreiten der Flüssigkeit (114) in dem Hohlraum (4, 34, 84, 94), wobei die Flüssigkeit (114) bis zu der zumindest einen Durchführung (3, 63, 73) fließt, und,
E) optional, elastisches Expandieren des Hohlraums (4, 34, 84, 94) durch Injizieren der Flüssigkeit (114).

13. Verfahren nach Anspruch 12, **dadurch gekennzeichnet, dass**
bei dem Verfahren keine medizinische Behandlung eines menschlichen oder tierischen Körpers erfolgt und/oder die Flüssigkeit (114) im Rahmen des Verfahrens nicht an einen menschlichen oder tierischen Körper abgegeben wird.

14. Verfahren nach einem der Ansprüche 12 oder 13, **dadurch gekennzeichnet, dass** in Schritt B) die Spitze der medizinischen Injektionskanüle (100) derart weit durch die obere Wandung (1, 31) gestochen wird, dass sie auf den Durchstechschutz (6, 86, 96) trifft, wobei eine Öffnung an der Spitze der medizinischen Injektionskanüle (100) dann in einem Innenraum des Hohlraums (4, 34, 84, 94) liegt, wobei der Innenraum des Hohlraums (4, 34, 84, 94) zwischen dem Durchstechschutz (6, 86, 96) und der oberen Wandung (1, 31) angeordnet ist.

15. Verfahren nach einem der Ansprüche 12 bis 14, **dadurch gekennzeichnet, dass** nach Schritt E) die folgenden Schritte F) und G) und gegebenenfalls H) erfolgen:
F) Herausziehen der medizinischen Injektionskanüle (100) aus dem Hohlraum (4, 34, 84, 94) und aus der oberen Wandung (1, 31) und
G) flüssigkeitsdichtes Verschließen der oberen Wandung (1, 31) durch gummielastische Rückstellung der oberen Wandung (1, 31), und optional
H) Komprimieren des Hohlraums (4, 34, 84, 94) aufgrund der elastischen Rückstellkraft des elastisch expandierten Hohlraums (4, 34, 84, 94) und Herausdrücken der Flüssigkeit (114) aus dem Hohlraum (4, 34, 84, 94) durch die zumindest eine Durchführung (3, 63, 73).
